# EUROPEAN PATENT APPLICATION

(11) **EP 4 844 774 A1**
(43) Date of publication of application: **25.11.2026**
(21) Application number: 26172513.9
(22) Date of filing: 15.04.2026
(51) Int. Cl.: G16H 10/40, G16B 20/20, G16B 40/00, G16B 45/00

(54) **METHOD AND APPARATUS FOR DETECTING CHROMOSOMAL COPY NUMBER VARIATION, DEVICE, MEDIUM, AND PRODUCT**

(30) Priority: 22.05.2025 CN 202510669935
(71) Applicant: GeneMind Biosciences Company Limited, Shenzhen City, Guangdong 518023 (CN)
(72) Inventor: LIU, Xianke, Shenzhen, 518023 (CN)
(74) Representative: Schiweck Weinzierl Koch Patentanwälte Partnerschaft mbB

(57) **Abstract**

The present disclosure discloses a method and an apparatus for detecting chromosomal copy number variation, a device, a medium, and a product. The method includes: obtaining sequencing depth data of a chromosome to be tested in a nucleic acid sample to be tested; determining a copy number type set based on the sequencing depth data and a normal distribution function set corresponding to the chromosome to be tested, where the normal distribution function set includes normal distribution functions respectively corresponding to a copy number deletion type, a copy number duplication type, and a copy number normal type, and the copy number type set includes labeled copy number types of a plurality of second nucleic acid windows; and combining, based on the copy number type set, the plurality of second nucleic acid windows to obtain at least one copy number variation region on the chromosome to be tested, where the second nucleic acid window is a first nucleic acid window corresponding to the chromosome to be tested, or a merged nucleic acid window obtained by merging a plurality of first nucleic acid windows based on a preset window number and a preset window step size, which improves the precision of detecting chromosomal copy number variation.

## Description

### TECHNICAL FIELD

The present disclosure relates to the technical field of biomedicine, and in particular, to a method and an apparatus for detecting chromosomal copy number variation, a device, a medium, and a product.

### BACKGROUND

In recent years, due to the discovery of fetal cell-free nucleic acids in maternal plasma and the development of next generation sequencing (NGS) technology, whole-genome low-depth sequencing has been applied to prenatal aneuploidy screening, and is mainly used for trisomy detection of chromosome 21, chromosome 18, and chromosome 13.

Studies have shown that, in a pregnant population, the proportion of fetuses carrying pathogenic chromosomal copy number variations (CNVs) may reach 1.6%-1.7%, which is much higher than the incidence of trisomy of about 0.2%. However, due to factors such as CNV regions being shorter than chromosomes, the low sequencing depth caused by sequencing costs, and a high sequencing signal-to-noise ratio, non-invasive testing for pregnant women is limited, resulting in a problem of significantly insufficient detection precision in CNV detection.

### SUMMARY

Embodiments of the present disclosure provide a method and an apparatus for detecting chromosomal copy number variation, a device, a medium, and a product to solve the problem of poor precision of CNV detection, so as to improve the accuracy of CNV detection.

According to an embodiment of the present disclosure, a method for detecting chromosomal copy number variation is provided, and the method includes:
obtaining sequencing depth data of a chromosome to be tested in a nucleic acid sample to be tested, where the sequencing depth data include first sequencing depths under a plurality of first nucleic acid windows corresponding to the chromosome to be tested;
determining a copy number type set based on the sequencing depth data and a normal distribution function set corresponding to the chromosome to be tested, where the normal distribution function set includes normal distribution functions respectively corresponding to a copy number deletion type, a copy number duplication type, and a copy number normal type, and the copy number type set includes labeled copy number types of a plurality of second nucleic acid windows; and
combining, based on the copy number type set, the plurality of second nucleic acid windows to obtain at least one copy number variation region on the chromosome to be tested,
where the second nucleic acid window is the first nucleic acid window, or the second nucleic acid window is a merged nucleic acid window obtained by merging the plurality of first nucleic acid windows based on a preset window number and a preset window step size, and the normal distribution function represents a probability density of a second sequencing depth corresponding to the second nucleic acid window varying with values of the second sequencing depth.

According to another embodiment of the present disclosure, an apparatus for detecting chromosomal copy number variation is provided, and the apparatus includes:
a sequencing depth data acquisition module, configured to obtain sequencing depth data of a chromosome to be tested in a nucleic acid sample to be tested, where the sequencing depth data include first sequencing depths under a plurality of first nucleic acid windows corresponding to the chromosome to be tested;
a copy number type set determination module, configured to determine a copy number type set based on the sequencing depth data and a normal distribution function set corresponding to the chromosome to be tested, where the normal distribution function set includes normal distribution functions respectively corresponding to a copy number deletion type, a copy number duplication type, and a copy number normal type, and the copy number type set includes labeled copy number types of a plurality of second nucleic acid windows; and
a copy number variation region determination module, configured to combine, based on the copy number type set, the plurality of second nucleic acid windows to obtain at least one copy number variation region on the chromosome to be tested,
where the second nucleic acid window is the first nucleic acid window, or the second nucleic acid window is a merged nucleic acid window obtained by merging the plurality of first nucleic acid windows based on a preset window number and a preset window step size, and the normal distribution function represents a probability density of a second sequencing depth corresponding to the second nucleic acid window varying with values of the second sequencing depth.

According to another embodiment of the present disclosure, an electronic device is provided, and the electronic device includes:
at least one processor; and
a memory in communication connection to the at least one processor, where
the memory stores one or more computer programs run by the at least one processor, and the one or more computer programs, when run by the at least one processor, cause the at least one processor to perform the method for detecting chromosomal copy number variation according to any one of the embodiments of the present disclosure.

According to another embodiment of the present disclosure, a computer-readable storage medium is provided, where the computer-readable storage medium stores one or more computer instructions, and the one or more computer instructions are used to, when executed by a processor, cause the processor to implement the method for detecting chromosomal copy number variation according to any one of the embodiments of the present disclosure.

According to another embodiment of the present disclosure, a computer program product is provided, wherein the computer program product includes one or more computer programs, and the one or more computer programs, when run by a processor, cause the processor to implement the method for detecting chromosomal copy number variation according to any one of the embodiments of the present disclosure.

According to the technical solutions of the embodiments, a copy number type set corresponding to second nucleic acid windows is determined based on a normal distribution function set corresponding to a chromosome to be tested and sequencing depth data corresponding to first nucleic acid windows, and at least one copy number variation region on the chromosome to be tested is obtained by combining a plurality of second nucleic acid windows based on the copy number type set. The normal distribution function set includes normal distribution functions respectively corresponding to a copy number deletion type, a copy number duplication type, and a copy number normal type. The second nucleic acid window is the first nucleic acid window, or is a merged nucleic acid window obtained by merging a plurality of first nucleic acid windows based on a preset window number and a preset window step size. Therefore, the objective of performing CNV detection based on window fragment features of chromosomes is achieved, the problem of poor precision of CNV detection is solved, and the accuracy of CNV detection is improved.

It should be understood that what is described in this section is not intended to identify key or critical features of the embodiments of the present disclosure, and it is also not intended to limit the scope of the present disclosure. Other features of the present disclosure will become apparent from the following description.

### BRIEF DESCRIPTION OF THE DRAWINGS

For clearer descriptions of the technical solutions according to the embodiments of the present disclosure, the drawings required to be used in the description of the embodiments are briefly introduced below. It is apparent that the drawings in the description below are only some embodiments of the present disclosure, and for those of ordinary skill in the art, other drawings may be obtained from the drawings without creative efforts.
FIG. 1 is a flowchart of a method for detecting chromosomal copy number variation according to an embodiment of the present disclosure;
FIG. 2 is a schematic diagram of a normal distribution function set according to an embodiment of the present disclosure;
FIG. 3 is a flowchart of another method for detecting chromosomal copy number variation according to an embodiment of the present disclosure;
FIG. 4 is a scatter plot of initial depth data and sequencing depth data according to an embodiment of the present disclosure;
FIG. 5 is a flowchart of a specific example of a method for detecting chromosomal copy number variation according to an embodiment of the present disclosure;
FIG. 6 is a schematic structural diagram of an apparatus for detecting chromosomal copy number variation according to an embodiment of the present disclosure; and
FIG. 7 is a schematic structural diagram of an electronic device according to an embodiment of the present disclosure.

### DETAILED DESCRIPTION

To enable those skilled in the art to better understand the solutions in the present disclosure, the technical solutions in the embodiments of the present disclosure will be clearly and completely described below with reference to the drawings in the embodiments of the present disclosure, and it is obvious that the described embodiments are only a part of the embodiments of the present disclosure but not all of them. Based on the embodiments of the present disclosure, all other embodiments obtained by those of ordinary skill in the art without creative efforts shall fall within the protection scope of the present disclosure.

It should be noted that the terms "first", "second", "third", "target", "reference", "initial", and the like in the specification and claims of the present disclosure and the above drawings are used to distinguish similar objects, and do not have to be used to describe a specific order or sequence. It should be understood that the data so used are interchangeable under appropriate circumstances, such that the embodiments of the present disclosure described herein are capable of being implemented in other sequences than those illustrated or described herein. Moreover, the terms "comprise", "include", and any variations thereof are intended to cover non-exclusive inclusion. For example, a process, method, system, product, or device including a series of steps or units is not necessarily limited to the explicitly listed steps or units, but may include other steps or units that are not explicitly listed or are inherent in the process, method, product, or device.

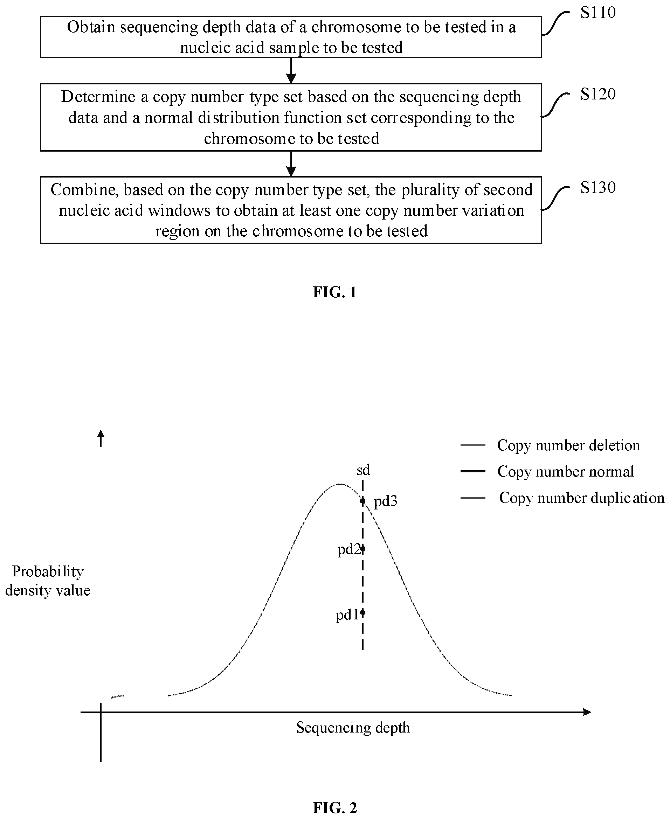

FIG. 1 is a flowchart of a method for detecting chromosomal copy number variation according to an embodiment of the present disclosure. The embodiment is applicable to a case of detecting chromosomal copy number variation. The method may be performed by an apparatus for detecting chromosomal copy number variation. The apparatus for detecting chromosomal copy number variation may be implemented in the form of hardware and/or software, and the apparatus for detecting chromosomal copy number variation may be configured in a terminal device. As shown in FIG. 1, the method includes the following steps.

In step S110, sequencing depth data of a chromosome to be tested in a nucleic acid sample to be tested are obtained.

Specifically, the nucleic acid sample to be tested is a cell-free DNA (cfDNA) sample, and the chromosome to be tested is any one pair of chromosomes among 23 pairs of human chromosomes used for detecting chromosomal copy number variation. The cfDNA sample refers to DNA fragments that are freely present in body fluids such as blood, cerebrospinal fluid, and urine and are not contained within cellular structures.

In an optional embodiment, the nucleic acid sample to be tested is extracted from a peripheral blood sample of a pregnant woman. Specifically, the peripheral blood sample contains maternal cells from the pregnant woman and the cfDNA sample. The cfDNA sample is mainly derived from apoptosis and necrosis of placental cells and active release of fetal cells.

By sequencing the nucleic acid sample to be tested, the sequencing depth data(namely, data regarding the depth of nucleic acid windows, or data on nucleic acid window depth) of the chromosome to be tested are obtained. As one implementation, the sequencing method includes: amplifying cell-free nucleic acids using a polymerase chain reaction (PCR) nucleic acid amplification instrument; constructing a library of the cell-free nucleic acids using a fetal chromosomal aneuploidy detection kit; and performing whole-genome sequencing on the cell-free nucleic acids using an NGS gene sequencer to obtain whole-genome sequencing data of the cell-free nucleic acids. Further, quality control conditions may be set to perform quality control on the whole-genome sequencing data, and sequencing data satisfying the quality control conditions, that is, sequencing data that pass quality control, are retained.

In some embodiments, the sequencing data that pass quality control is input into alignment software to align nucleic acid sequences to a human reference genome, and an alignment result data file is generated for each sample. The alignment result data file is input into a sequence counting tool to further filter the alignment result data and remove PCR duplicate data. Based on positions where the nucleic acid sequences are aligned to the genome (also referred to as "preset window positions"), the number of sequences at respective positions is counted. The number of nucleic acid sequences at a position corresponds to the depth at the position. In this way, a sequencing depth file, i.e., sequencing depth data, of a sample is obtained.

In the embodiment, the sequencing depth data include first sequencing depths under a plurality of first nucleic acid windows corresponding to the chromosome to be tested.

The first nucleic acid window represents a nucleic acid fragment corresponding to the chromosome to be tested in the human reference genome. In an optional embodiment, the method further includes: obtaining chromosome nucleic acid data corresponding to the chromosome to be tested from reference genome nucleic acid data corresponding to the human reference genome; and dividing, according to a preset window length, the chromosome nucleic acid data to obtain a plurality of first nucleic acid windows.

Illustratively, the source of the human reference genome may be version GRCH36, version GRCH37, or version GRCh38 of a database of the National Center for Biotechnology Information (NCBI), or version hg18, version hg19, or version hg38 of a database of the University of California, Santa Cruz (UCSC), or the like. The source of the human reference genome is not limited herein and may be specifically customized according to actual requirements.

Specifically, the reference genome nucleic acid data represent a collection of information obtained by sequencing the human reference genome, and the collection of information consists of base sequence information, gene expression information, functional annotations, and the like. The chromosome nucleic acid data are nucleic acid sequences corresponding to the chromosome to be tested in the human reference genome.

Illustratively, the preset window length is 20 kbp, which is, however, not limited to the example.

In an optional embodiment, dividing, according to the preset window length, the chromosome nucleic acid data to obtain the plurality of first nucleic acid windows corresponding to the chromosome to be tested includes: dividing, according to the preset window length, the chromosome nucleic acid data to obtain a window division result; and deleting nucleic acid windows in the window division result that do not contain known bases to obtain the plurality of first nucleic acid windows corresponding to the chromosome to be tested.

Specifically, the window division result includes a plurality of nucleic acid windows having the preset window length. The nucleic acid windows in the window division result are traversed, and if a nucleic acid window does not contain known bases, it indicates that the nucleic acid window consists entirely of unknown bases, and the nucleic acid window is deleted from the window division result.

The advantage of such an arrangement is that noise interference caused by nucleic acid windows consisting entirely of unknown bases is avoided, thereby further improving the accuracy of CNV detection.

Specifically, the sequencing depth refers to the number of sequences in the nucleic acid sample to be tested that can be uniquely aligned to a certain region in the human reference genome.

In an optional embodiment, obtaining the sequencing depth data of the chromosome to be tested in the nucleic acid sample to be tested includes: obtaining chromosome sequencing data corresponding to the chromosome to be tested in genomic sequencing data of the nucleic acid sample to be tested; separately performing sequence alignment between the chromosome sequencing data and the plurality of first nucleic acid windows corresponding to the chromosome to be tested to obtain sequence alignment results; and determining the sequencing depth data based on the sequence alignment results.

Specifically, the genomic sequencing data are nucleic acid sequence data obtained by performing whole-genome sequencing on the nucleic acid sample to be tested, representing a collection of information consisting of base sequence information, gene expression information, functional annotations, and the like obtained by sequencing the chromosome to be tested. The chromosome sequencing data are nucleic acid sequences corresponding to the chromosome to be tested in the nucleic acid sample to be tested.

Based on the above embodiments, optionally, the detection method further includes: performing polymerase chain reaction (PCR) amplification on the nucleic acid sample to be tested using a PCR nucleic acid amplification instrument, and then performing sample preprocessing to obtain a nucleic acid library; and performing whole-genome sequencing on the nucleic acid library to obtain genomic sequencing data of the nucleic acid sample to be tested.

Illustratively, the sample preprocessing includes, but is not limited to, cfDNA fragmentation, end repair, adaptor ligation, quality control, quantitative analysis, and the like. An Illumina library preparation kit is used to construct the nucleic acid library based on the amplified nucleic acid sample to be tested. Sequencing technologies used for whole-genome sequencing include, but are not limited to, next generation sequencing technology, nanopore sequencing technology, third-generation sequencing technology, or the like.

In an optional embodiment, the detection method further includes: performing data quality control on the genomic sequencing data of the nucleic acid sample to be tested to obtain genomic sequencing data that pass quality control. The data quality control referred to in the embodiments of the present application refers to filtering sequencing data so as to remove sequencing data that do not satisfy quality control conditions. For example, sequencing data that do not satisfy quality control conditions include, but are not limited to, sequencing data in which a proportion of unknown bases N is excessively high, a proportion of low-quality bases is excessively high, or a remaining sequence length after adaptor removal is excessively short. Illustratively, the quality control tool used for the data quality control may be a fastp tool, a Trimmomatic tool, or a FastQC tool. The quality control tools used herein are not limited thereto and may be customized according to actual requirements.

Illustratively, alignment tools used for sequence alignment include, but are not limited to, Tmap, BWA, SOAP, samtools, or the like.

Specifically, determining the sequencing depth data based on the sequence alignment results includes: performing a PCR duplicate removal operation on the sequence alignment results to remove duplicates introduced by PCR amplification, and determining the sequencing depth data based on the sequence alignment results after removal of PCR duplicates.

The duplicates herein refer to sequences that, after alignment to the human reference genome, have completely identical comparison parameters including chromosome, chromosomal position, length, direction, and base sequence, and only one sequence is retained to reduce bias and error caused by PCR amplification.

In an optional embodiment, determining the sequencing depth data based on the sequence alignment results includes: performing correction processing on sequencing depths in the sequence alignment results, and determining the sequencing depth data based on the sequence alignment results after correction processing.

The correction processing includes at least one of effective base length correction, outlier correction, mappability correction, and guanine and cytosine (GC) base content correction. The mappability value may be used to characterize the alignment capability of an alignment tool to correctly align chromosome sequencing data to nucleic acid windows of the human reference genome. The mappability correction refers to performing local polynomial regression fitting correction on the sequencing depth based on the mappability value. Since the sequencing depths corresponding to sequence alignment results with high GC content or low GC content tend to be lower than the sequencing depths corresponding to sequence alignment results in which GC content and AT content differ slightly or tend to be consistent, the GC base content correction refers to performing standardized correction or local polynomial regression fitting correction on the sequencing depth based on the GC content corresponding to the sequence alignment result.

The advantage of such an arrangement is that error interference on the sequencing depth of the target chromosome caused by different effective base lengths, different outliers, different mappability values, and different GC contents can be eliminated, thereby further improving the accuracy of CNV detection.

In step S120, a copy number type set is determined based on the sequencing depth data and a normal distribution function set corresponding to the chromosome to be tested.

In the embodiment, the normal distribution function set includes normal distribution functions respectively corresponding to a copy number deletion type, a copy number duplication type, and a copy number normal type.

The copy number deletion type indicates that the copy number of a certain region or a certain gene on a chromosome is lower than the normal level; the copy number duplication type indicates that the copy number of a certain region or a certain gene on a chromosome is higher than the normal level; and the copy number normal type indicates that the copy number of a certain region or a certain gene on a chromosome is equal to the normal level.

In the embodiment, the normal distribution function represents a probability density of a second sequencing depth corresponding to a second nucleic acid window varying with values of the second sequencing depth, and the copy number type set includes labeled copy number types of a plurality of second nucleic acid windows.

In a specific embodiment, the second nucleic acid window is the first nucleic acid window, or the second nucleic acid window is a merged nucleic acid window obtained by merging the plurality of first nucleic acid windows based on a preset window number and a preset window step size.

Specifically, the preset window number represents the number of first nucleic acid windows included in the second nucleic acid window, and the preset window step size represents the number of first nucleic acid windows overlapping between two adjacent second nucleic acid windows.

In an optional embodiment, the preset window number is twice the preset window step size. Illustratively, the preset window number is 10, and the preset window step size is 5.

In an optional embodiment, determining the copy number type set based on the sequencing depth data and the normal distribution function set corresponding to the chromosome to be tested includes: obtaining a normal distribution function set in a first iteration process; determining, for each second nucleic acid window, the second sequencing depth corresponding to the second nucleic acid window based on the sequencing depth data, and determining, based on the normal distribution function set and the second sequencing depth, probability density values respectively corresponding to the copy number deletion type, the copy number duplication type, and the copy number normal type of the second nucleic acid window, and using the copy number type corresponding to the maximum probability density value as the labeled copy number type of the second nucleic acid window; updating, based on the second sequencing depth of at least one second nucleic acid window corresponding to the copy number normal type, the normal distribution function set to obtain a normal distribution function set of a next iteration process; returning to execute the step of determining, for each second nucleic acid window, the second sequencing depth corresponding to the second nucleic acid window based on the sequencing depth data; and until the number of the second nucleic acid windows corresponding to the copy number normal type in the chromosome to be tested converges, determining the copy number type set based on the labeled copy number type of each second nucleic acid window in a current iteration process.

Specifically, standard deviations respectively corresponding to the three normal distribution functions in the normal distribution function set are the same. In the first iteration process, the standard deviations respectively corresponding to the three normal distribution functions in the normal distribution function set are preset values. Illustratively, the preset value may be 0.1, which is, however, not limited to the example.

In an optional embodiment, determining the second sequencing depth corresponding to the second nucleic acid window based on the sequencing depth data includes: when the second nucleic acid window is the first nucleic acid window, determining a first sequencing depth corresponding to the second nucleic acid window in the sequencing depth data as the second sequencing depth corresponding to the second nucleic acid window.

In another optional embodiment, determining the second sequencing depth corresponding to the second nucleic acid window based on the sequencing depth data includes: when the second nucleic acid window is a merged nucleic acid window, obtaining first sequencing depths of a preset window number of first nucleic acid windows corresponding to the second nucleic acid window in the sequencing depth data; and using an average value of the preset window number of first sequencing depths as the second sequencing depth corresponding to the second nucleic acid window.

FIG. 2 is a schematic diagram of a normal distribution function set according to an embodiment of the present disclosure Specifically, the green curve, the black curve, and the purple curve respectively represent normal distribution functions in the normal distribution function set corresponding to the copy number deletion type, the copy number normal type, and the copy number duplication type; the horizontal axis represents sequencing depths, and the vertical axis represents probability density values.

Taking FIG. 2 as an example, the second sequencing depth sd is substituted into the normal distribution function corresponding to the copy number deletion type (green curve) to obtain a probability density value pd1 corresponding to the copy number deletion type; the second sequencing depth sd is substituted into the normal distribution function corresponding to the copy number normal type (black curve) to obtain a probability density value pd2 corresponding to the copy number normal type; and the second sequencing depth sd is substituted into the normal distribution function corresponding to the copy number duplication type (purple curve) to obtain a probability density value pd3 corresponding to the copy number duplication type. By comparing the probability density value pd1, the probability density value pd2, and the probability density value pd3, when the probability density value pd3 is the largest, the copy number duplication type is used as the labeled copy number type of the second nucleic acid window.

In an optional embodiment, updating, based on the second sequencing depth of at least one second nucleic acid window corresponding to the copy number normal type, the normal distribution function set to obtain the normal distribution function set of the next iteration process includes: determining a standard deviation corresponding to the second sequencing depth of at least one second nucleic acid window corresponding to the copy number normal type; and updating, based on the standard deviation, the normal distribution function set to obtain the normal distribution function set of the next iteration process.

Specifically, the standard deviations corresponding to the three normal distribution functions in the normal distribution function set are respectively replaced based on the determined standard deviation, so as to obtain the normal distribution function set of the next iteration process.

In an optional embodiment, in the normal distribution function set, the mean value of the normal distribution function corresponding to the copy number normal type is 1, the mean value of the normal distribution function corresponding to the copy number deletion type is $1 - \frac{FF}{2}$, and the mean value of the normal distribution function corresponding to the copy number duplication type is $1 + \frac{FF}{2}$, where *FF* denotes a placental-derived nucleic acid fraction corresponding to the nucleic acid sample to be tested.

In the embodiment, the first sequencing depth is a sequencing depth after normalization processing. Illustratively, the normalization algorithm used in the normalization processing is a median value normalization algorithm.

Specifically, the placental-derived nucleic acid fraction represents the proportion of nucleic acid content derived from the placenta in the nucleic acid sample to be tested relative to the total nucleic acid content of the nucleic acid sample to be tested. Illustratively, the placental-derived nucleic acid fraction may also be referred to as placental DNA or a placental DNA fraction.

In some examples, under a condition that the placental-derived nucleic acid fraction FF remains unchanged, statistical results of multiple repeated detection experiments on nucleic acid samples show that the sequencing depth of a normal diploid sample satisfies a normal distribution function with a mean value of 1, the sequencing depth of a copy number deletion sample satisfies a normal distribution function with a mean value of $1 - \frac{FF}{2}$, and the normalized sequencing depth of a copy number duplication sample satisfies a normal distribution function with a mean value of $1 + \frac{FF}{2}$.

In another optional embodiment, in the normal distribution function set, the mean value of the normal distribution function corresponding to the copy number normal type is *mean,* the mean value of the normal distribution function corresponding to the copy number deletion type is $mean - \frac{FF}{2}$*,* and the mean value of the normal distribution function corresponding to the copy number duplication type is $mean + \frac{FF}{2}$*,* where *mean* denotes a median value of the second sequencing depths respectively corresponding to a plurality of second nucleic acid windows.

The advantage of setting the median value to replace 1 lies in that, due to influences of experimental technical differences, characteristics of samples themselves, and other factors, the distribution center of the second sequencing depth may fluctuate significantly, and the median value can more accurately reflect the central tendency of the second sequencing depth, such that the normal distribution function better fits actual data distributions of different nucleic acid samples to be tested, thereby further improving accuracy and stability of CNV detection.

Based on the above embodiments, optionally, the detection method further includes: determining alignment feature data based on the genomic sequencing data of the nucleic acid sample to be tested and the reference genome nucleic acid data of a human reference genome; and inputting the alignment feature data into a pre-trained nucleic acid dosage prediction model to obtain, as an output, the placental-derived nucleic acid fraction of the nucleic acid sample to be tested.

Illustratively, the alignment feature data include, but are not limited to, an aligned chromosome, an aligned gene on the chromosome, an aligned position on the chromosome, and gene annotation, which are, however, not limited to the examples.

In step S130, based on the copy number type set, the plurality of second nucleic acid windows are combined to obtain at least one copy number variation region on the chromosome to be tested.

In an optional embodiment, the copy number variation region satisfies the following combining conditions:
1) the labeled copy number types of a first second nucleic acid window and a last second nucleic acid window in the copy number variation region are both copy number abnormal types;
2) the number of second nucleic acid windows included in the copy number variation region whose labeled copy number types are the copy number abnormal types is not less than a preset number threshold;
3) the number of second nucleic acid windows included in the copy number variation region whose labeled copy number types correspond to the copy number normal type is not greater than a preset number threshold; and
4) a plurality of copy number abnormal types corresponding to the copy number variation region are either the copy number deletion type or the copy number duplication type.

Illustratively, the preset number threshold may be 6, which is, however, not limited to the example.

According to the technical solutions of the embodiments, a copy number type set corresponding to second nucleic acid windows is determined based on a normal distribution function set corresponding to a chromosome to be tested and sequencing depth data corresponding to first nucleic acid windows, and at least one copy number variation region on the chromosome to be tested is obtained by combining a plurality of second nucleic acid windows based on the copy number type set. The normal distribution function set includes normal distribution functions respectively corresponding to a copy number deletion type, a copy number duplication type, and a copy number normal type. The second nucleic acid window is the first nucleic acid window, or is a merged nucleic acid window obtained by merging a plurality of first nucleic acid windows based on a preset window number and a preset window step size. Therefore, the objective of performing CNV detection based on window fragment features of chromosomes is achieved, the problem of poor precision of CNV detection is solved, and the accuracy of CNV detection is improved.

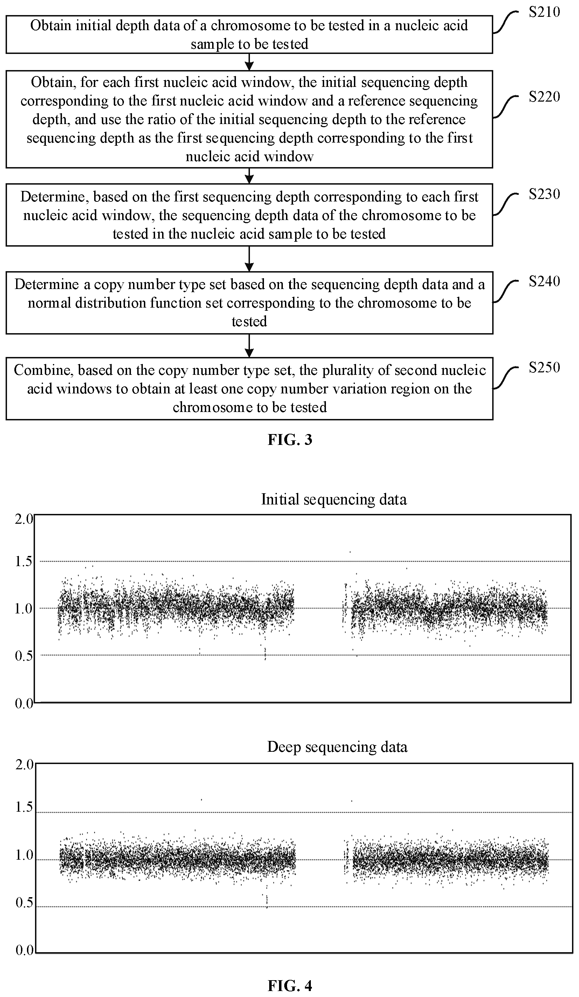

FIG. 3 is a flowchart of another method for detecting chromosomal copy number variation according to an embodiment of the present disclosure. In the embodiment, the step of "obtaining sequencing depth data of a chromosome to be tested in a nucleic acid sample to be tested" in the above embodiment is further refined. As shown in FIG. 3, the method includes the following steps.

In step S210, initial depth data of a chromosome to be tested in a nucleic acid sample to be tested is obtained.

In the embodiment, the initial depth data include initial sequencing depths under a plurality of first nucleic acid windows corresponding to the chromosome to be tested.

Specifically, the method for obtaining the initial depth data is identical or similar to the method for obtaining the sequencing depth data provided in the above embodiments, which will not be repeated herein.

In step S220, for each first nucleic acid window, the initial sequencing depth corresponding to the first nucleic acid window and a reference sequencing depth are obtained, and the ratio of the initial sequencing depth to the reference sequencing depth is used as the first sequencing depth corresponding to the first nucleic acid window.

In the embodiment, the reference sequencing depth represents an average value of third sequencing depths respectively corresponding to the nucleic acid sample to be tested and at least one third nucleic acid window in a reference nucleic acid window set, and the third nucleic acid window is a nucleic acid window corresponding to other chromosomes except the chromosome to be tested. Specifically, the reference nucleic acid window set represents a window set of third nucleic acid windows having sequencing depth similarity with the first nucleic acid window.

In an optional embodiment, the detection method further includes: obtaining target depth data corresponding to the chromosome to be tested and reference depth data corresponding to the other chromosomes from a negative nucleic acid sample set; determining, for each first nucleic acid window, depth distance data based on the reference depth data and the target depth sequence corresponding to the first nucleic acid window, and screening, based on the depth distance data, a plurality of third nucleic acid windows to obtain the reference nucleic acid window set corresponding to the first nucleic acid window.

The target depth data include target depth sequences under a plurality of first nucleic acid windows corresponding to the chromosome to be tested, and the target depth sequences include negative sequencing depths of at least one negative nucleic acid sample in the negative nucleic acid sample set under the first nucleic acid window. The negative nucleic acid sample is a nucleic acid sample without a CNV region.

Illustratively, the target depth data are denoted as *SD_{g} =* {*sd*₁ *sd*₂*, ..., sdₙ*}*,* where *sd*₁ denotes the target depth sequence corresponding to the first first nucleic acid window, *sd*₂ denotes the target depth sequence corresponding to the second first nucleic acid window, and *sdₙ* denotes the target depth sequence corresponding to the nth first nucleic acid window.

Taking the target depth sequence *sdₙ* as an example, when the chromosome to be tested is a non-X chromosome, ${sd}_{n} = \left\{{sd}_{n}^{1},{sd}_{n}^{2},…,{sd}_{n}^{s}\right\}$; and when the chromosome to be tested is an X chromosome, ${sd}_{n} = \left\{{sd}_{n}^{1},{sd}_{n}^{2},…,{sd}_{n}^{q}\right\}$, where ${sd}_{n}^{1}$ denotes the negative sequencing depth of the first negative nucleic acid sample under the nth first nucleic acid window, ${sd}_{n}^{2}$ denotes the negative sequencing depth of the second negative nucleic acid sample under the nth first nucleic acid window, ${sd}_{n}^{s}$ denotes the negative sequencing depth of the sth negative nucleic acid sample under the nth first nucleic acid window, ${sd}_{n}^{q}$ denotes the negative sequencing depth of the *q*th negative nucleic acid sample under the nth first nucleic acid window, *s* denotes the total number of negative nucleic acid samples in the negative nucleic acid sample set, *q* denotes the number of negative nucleic acid samples corresponding to female fetuses in the negative nucleic acid sample set, and *q* < *s*.

Particularly, since only negative nucleic acid samples corresponding to male fetuses have sequencing depth of a Y chromosome, and the sequencing depth varies with the placental-derived nucleic acid fraction, the chromosome to be tested in the embodiment is not a Y chromosome.

In the embodiment, the reference depth data include reference depth sequences under a plurality of third nucleic acid windows corresponding to other chromosomes, and the reference depth sequences include negative sequencing depths of at least one negative nucleic acid sample under the third nucleic acid window. Specifically, the number of other chromosomes corresponding to the reference depth data is one or more.

In the embodiment, the depth distance data include depth distances respectively corresponding to the target depth sequence and at least one reference depth sequence. Specifically, the depth distance represents a similarity degree between the target depth sequence and the reference depth sequence.

Illustratively, a depth distance *D*(*i, j*)² corresponding to the ith target depth sequence and the *j*th reference depth sequence satisfies the following formula: $D {\left(i, j\right)}^{2} = {\sum }_{m = 1}^{n} {\left({sd}_{i}^{m}-{sd}_{j}^{m ′}\right)}^{2}$ where when the chromosome to be tested is a non-X chromosome, n = *s*, and when the chromosome to be tested is an X chromosome, *n* = *q.* ${sd}_{i}^{m}$ denotes the negative sequencing depth of the *m*th negative nucleic acid sample under the ith first nucleic acid window, and ${sd}_{j}^{m ′}$ denotes the negative sequencing depth of the mth negative nucleic acid sample under the *j*th third nucleic acid window.

In an optional embodiment, screening, based on the depth distance data, the plurality of third nucleic acid windows to obtain the reference nucleic acid window set corresponding to the first nucleic acid window includes: retaining, based on the depth distance data, third nucleic acid windows satisfying a specified window number in ascending order to obtain an initial nucleic acid window set corresponding to the first nucleic acid window; and performing data cleaning on the initial nucleic acid window set to obtain the reference nucleic acid window set corresponding to the first nucleic acid window.

Illustratively, the specified window number may be 300 or 400, which is, however, not limited to the examples.

In an optional embodiment, performing data cleaning on the initial nucleic acid window set to obtain the reference nucleic acid window set corresponding to the first nucleic acid window includes: when the initial nucleic acid window set includes two adjacent third nucleic acid windows, obtaining depth distances respectively corresponding to the two third nucleic acid windows in the depth distance data; and removing, from the initial nucleic acid window set, the third nucleic acid window corresponding to the larger depth distance to obtain the reference nucleic acid window set corresponding to the first nucleic acid window.

Specifically, the two adjacent third nucleic acid windows indicate that the two third nucleic acid windows are located on the same chromosome and are adjacent in position on the chromosome.

Illustratively, assuming that in the initial nucleic acid window set, if the depth distance *D*(*i, j*)² corresponding to the *i*th target depth sequence and the *j*th reference depth sequence is greater than the depth distance *D*(*i, j* + 1)² corresponding to the ith target depth sequence and the *j* + 1th reference depth sequence, the *j*th third nucleic acid window is removed from the initial nucleic acid window set; and if the depth distance *D*(*i, j*)² corresponding to the ith target depth sequence and the *j*th reference depth sequence is less than the depth distance *D*(*i,j* + 1)² corresponding to the *i*th target depth sequence and the *j* + 1th reference depth sequence, the *j* + 1th third nucleic acid window is removed from the initial nucleic acid window set.

In the nucleic acid sample to be tested, two adjacent third nucleic acid windows may both be located within the same copy number variation region, which may introduce significant noise interference to the accuracy of the first sequencing depth obtained by performing internal reference correction on the initial sequencing depth. In the embodiment, by screening adjacent third nucleic acid windows in the initial nucleic acid window set, the accuracy of CNV detection is further improved.

In an optional embodiment, performing data cleaning on the initial nucleic acid window set to obtain the reference nucleic acid window set corresponding to the first nucleic acid window includes: determining coefficient of variation data based on a reference depth sequence of each third nucleic acid window in the initial nucleic acid window set, where the coefficient of variation data include a coefficient of variation of each third nucleic acid window; determining an outlier interval based on the coefficient of variation data; and removing, from the initial nucleic acid window set, the third nucleic acid window corresponding to a coefficient of variation that does not satisfy the outlier interval in the coefficient of variation data to obtain the reference nucleic acid window set corresponding to the first nucleic acid window.

Specifically, the coefficient of variation is the ratio of the standard deviation to the mean value corresponding to the reference depth sequence, and is used to measure the dispersion of the reference depth sequence. Illustratively, the coefficient of variation *CVᵢ* of the ith third nucleic acid window in the initial nucleic acid window set satisfies the following formula: ${CV}_{i} = \frac{{σ}_{i}}{{μ}_{i}}$ where *σᵢ* denotes the standard deviation corresponding to the reference depth sequence of the *i*th third nucleic acid window, and *µᵢ* denotes the mean value corresponding to the reference depth sequence of the *i*th third nucleic acid window.

Specifically, coefficients of variation in the coefficient of variation data are sorted in ascending order, a coefficient of variation at a position of the first 25% in the sorting result is taken as a lower quartile, denoted as *Q*₁*,* and a coefficient of variation at a position of the first 75% in the sorting result is taken as an upper quartile, denoted as *Q*₃. Correspondingly, the interquartile range *IRQ =* |*Q*₁ - *Q*₃|*.*

Illustratively, the maximum value corresponding to the outlier interval *Up_threhold = Q*₃ *+* 1.5 × *IRQ,* and the minimum value corresponding to the outlier interval *Down_threhold = Q*₁ *-* 1.5 × *IRQ,* which are, however, not limited to the examples.

The advantage of such an arrangement is that the stability of sequencing depths corresponding to the third nucleic acid windows in the reference nucleic acid window set is ensured, thereby reducing the influence of fluctuations of sequencing depths of the third nucleic acid windows on the first sequencing depth, and further ensuring the stability of CNV detection.

Based on the above embodiments, optionally, the detection method further includes: after performing data cleaning on the initial nucleic acid window set, if a difference value between the specified nucleic acid window number and the window number corresponding to the initial nucleic acid window set is greater than 0, retaining, based on the depth distance data and in ascending order, the remaining third nucleic acid windows in an amount equal to the difference value, and adding the third nucleic acid windows in an amount equal to the difference value to the initial nucleic acid window set; and returning to perform the step of data cleaning on the initial nucleic acid window set until the difference value is equal to 0, and using the initial nucleic acid window set as the reference nucleic acid window set corresponding to the first nucleic acid window.

The advantage of such an arrangement is that the case in which the number of third nucleic acid windows in the reference nucleic acid window set is excessively small due to the data cleaning operation is avoided, thereby ensuring the accuracy of the first sequencing depth and further improving the accuracy of CNV detection.

In step S230, based on the first sequencing depth corresponding to each first nucleic acid window, the sequencing depth data of the chromosome to be tested in the nucleic acid sample to be tested are determined.

FIG. 4 is a scatter plot of initial depth data and sequencing depth data according to an embodiment of the present disclosure. Specifically, taking chromosome 1 as an example in FIG. 4, the horizontal axis represents regions on chromosome 1, and the vertical axis represents sequencing depths. In FIG. 4, the upper panel shows a scatter plot of the initial depth data, and the lower panel shows a scatter plot of the sequencing depth data.

As can be seen from FIG. 4, the sequencing depth data exhibit a higher degree of concentration compared with the initial depth data, indicating that the dispersion of the sequencing depth data is reduced and the stability of the sequencing depth is higher.

In step S240, a copy number type set is determined based on the sequencing depth data and a normal distribution function set corresponding to the chromosome to be tested.

In step S250, based on the copy number type set, the plurality of second nucleic acid windows are combined to obtain at least one copy number variation region on the chromosome to be tested.

Steps S240 to S250 in the embodiment are identical or similar to steps S120 to S130 shown in FIG. 1 in the above embodiments, which will not be repeated herein.

Based on the above embodiments, optionally, after combining, based on the copy number type set, the plurality of second nucleic acid windows to obtain at least one copy number variation region on the chromosome to be tested, the detection method further includes: obtaining, for each copy number variation region, copy number parameter data corresponding to the copy number variation region; and in response to the copy number parameter data satisfying a parameter screening condition, using the copy number variation region as a final copy number variation region.

In the embodiment, the copy number parameter data include at least one of a copy number abnormal rate, a window density, and a region nucleic acid length.

The copy number abnormal rate represents the ratio of the number of second nucleic acid windows having the copy number abnormal type in the copy number variation region to the total number of second nucleic acid windows in the copy number variation region, the window density represents the number of first nucleic acid windows included within a unit nucleic acid length, and the copy number abnormal type is the copy number deletion type or the copy number duplication type.

Specifically, the unit nucleic acid length is greater than the window length of the first nucleic acid window. Illustratively, the unit nucleic acid length may be 1 M, which is, however, not limited to the example.

In an optional embodiment, the parameter screening condition includes at least one of the following: the copy number abnormal rate being greater than a preset abnormal rate threshold, the window density being greater than a preset density threshold, and the region nucleic acid length being greater than a preset length threshold.

A higher window density indicates a higher likelihood that the copy number variation region is a reliable copy number variation region; conversely, a lower window density indicates a lower likelihood that the copy number variation region is a reliable copy number variation region.

The advantage of providing the parameter screening condition is that the confidence of the copy number variation region is improved, and the accuracy of CNV detection is further improved.

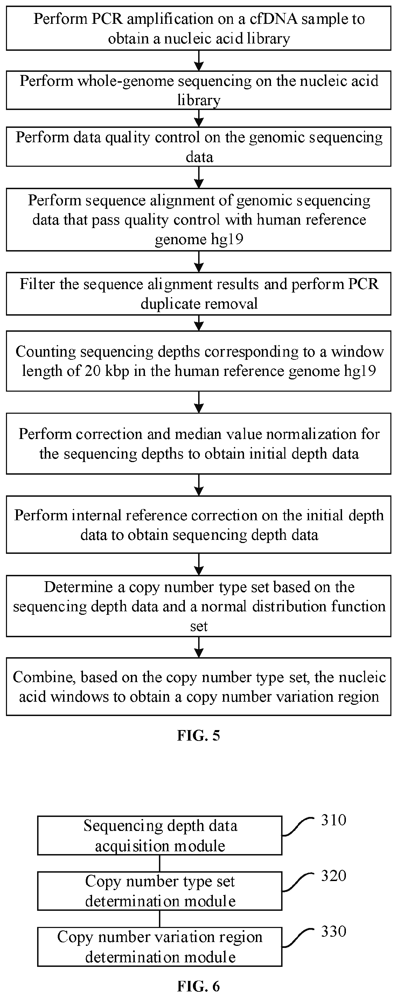

FIG. 5 is a flowchart of a specific example of a method for detecting chromosomal copy number variation according to an embodiment of the present disclosure. Specifically, a cfDNA sample is subjected to PCR amplification to obtain a nucleic acid library, whole-genome sequencing is performed on the nucleic acid library to obtain genomic sequencing data, and data quality control is performed on the genomic sequencing data. Illustratively, the quality control tool used for the data quality control may be a fastp tool, a Trimmomatic tool, or a FastQC tool. The genomic sequencing data that pass quality control are aligned to reference genome nucleic acid data of a human reference genome hg19, and the obtained sequence alignment results are filtered and subjected to PCR duplicate removal.

Sequencing depths corresponding to a window length of 20 kbp in the reference genome nucleic acid data of the human reference genome hg19 are counted, and correction processing and median value normalization are performed on the sequencing depths to obtain initial depth data corresponding to each chromosome. The correction processing may include effective base length correction, outlier correction, mappability correction, GC base content correction, and the like. For each chromosome, internal reference correction is performed on the initial depth data corresponding to the chromosome to obtain sequencing depth data. A copy number type set is determined based on the sequencing depth data and a normal distribution function set, and 200 kbp windows are combined based on the copy number type set to obtain a copy number variation region.

Since CNV regions are typically a small portion of a chromosome, compared with the entire chromosome, the variation in sequencing depth thereof is smaller but exhibits greater fluctuation. The difference between the sequencing depth of CNV regions and the sequencing depth of negative regions is also small. Therefore, the degree of fluctuation in sequencing depth is a key factor affecting the accuracy of CNV detection. Meanwhile, in different nucleic acid samples, CNV regions may occur at numerous possible positions on chromosomes and exhibit significant variability. Thus, it is difficult to construct an external parameter set as a correction reference for sequencing depth, and the correction effect cannot be ensured.

According to the technical solutions of the embodiments, for each first nucleic acid window, the initial sequencing depth corresponding to the first nucleic acid window is corrected based on a reference nucleic acid window set corresponding to the first nucleic acid window to obtain a first sequencing depth. The reference nucleic acid window set includes third sequencing depths under third nucleic acid windows corresponding to other chromosomes except the chromosome to be tested in the nucleic acid sample to be tested. Therefore, the objective of internal reference correction of the initial sequencing depth of the nucleic acid sample to be tested is achieved, the degree of fluctuation of deep sequencing data is reduced, and thus the accuracy of CNV detection is further improved.

### Example I

Genomic sequencing data of 356 negative nucleic acid samples were used, which were obtained from a NIPT plus assay based on next generation sequencing approved for medical device use, where 196 samples were negative nucleic acid samples from male fetuses and 160 samples were negative nucleic acid samples from female fetuses.

The following Table 1 shows CNV detection results of the 356 negative nucleic acid samples provided in Example I of the present disclosure.

**Table 1**

| No. | True result | Detection result | Result comparison and determination |
|---|---|---|---|
| a215 | Negative | del(chr7:q11.21,62480001-66880001,4.40Mb,16.55%) | False positive |
| a226 | Negative | dup(chr10:q26.13,124660001-126880001,2.22Mb,13.22%) | False positive |
| a314 | Negative | del(chr19:p13.2,9520001-12220001,2.70Mb,13.39%) | False positive |
| a323 | Negative | del(chr7:q11.21,62060001-64240001,2.18Mb,16.01%) | False positive |
| a334 | Negative | del(chr16:p11.2,28200001-31540001,3.34Mb,14.90%) | False positive |
| b054 | Negative | del(chr7:q11.23,73640001-75980001,2.34Mb,13.82%) | False positive |
| b101 | Negative | del(chr16:p12.1-p11.2,27700001-29780001,2.08Mb,12.13 %) | False positive |
| b120 | Negative | del(chr16:p11.2,28860001-31880001,3.02Mb,9.22%) | False positive |
| b124 | Negative | dup(chr16:p13.11-p12.3,15920001-17980001,2.06Mb,13.1 4%) | False positive |
| b200 | Negative | dup(chr2:q21.1-q21.2,131480001-133800001,2.32Mb,11.9 1%) | False positive |
| a003 | Negative | Negative | True negative |
| a004 | Negative | Negative | True negative |
| a006 | Negative | Negative | True negative |
| a008 | Negative | Negative | True negative |
| a009 | Negative | Negative | True negative |
| a010 | Negative | Negative | True negative |
| a011 | Negative | Negative | True negative |
| a012 | Negative | Negative | True negative |
| a014 | Negative | Negative | True negative |
| a015 | Negative | Negative | True negative |
| a016 | Negative | Negative | True negative |
| a017 | Negative | Negative | True negative |
| a018 | Negative | Negative | True negative |
| a019 | Negative | Negative | True negative |
| a021 | Negative | Negative | True negative |
| a022 | Negative | Negative | True negative |
| a023 | Negative | Negative | True negative |
| a025 | Negative | Negative | True negative |
| a027 | Negative | Negative | True negative |
| a028 | Negative | Negative | True negative |
| a029 | Negative | Negative | True negative |
| a030 | Negative | Negative | True negative |
| a031 | Negative | Negative | True negative |
| a032 | Negative | Negative | True negative |
| a035 | Negative | Negative | True negative |
| a037 | Negative | Negative | True negative |
| a038 | Negative | Negative | True negative |
| a039 | Negative | Negative | True negative |
| a041 | Negative | Negative | True negative |
| a042 | Negative | Negative | True negative |
| a043 | Negative | Negative | True negative |
| a044 | Negative | Negative | True negative |
| a045 | Negative | Negative | True negative |
| a048 | Negative | Negative | True negative |
| a052 | Negative | Negative | True negative |
| a053 | Negative | Negative | True negative |
| a054 | Negative | Negative | True negative |
| a055 | Negative | Negative | True negative |
| a058 | Negative | Negative | True negative |
| a060 | Negative | Negative | True negative |
| a061 | Negative | Negative | True negative |
| a062 | Negative | Negative | True negative |
| a063 | Negative | Negative | True negative |
| a064 | Negative | Negative | True negative |
| a066 | Negative | Negative | True negative |
| a068 | Negative | Negative | True negative |
| a069 | Negative | Negative | True negative |
| a070 | Negative | Negative | True negative |
| a071 | Negative | Negative | True negative |
| a072 | Negative | Negative | True negative |
| a075 | Negative | Negative | True negative |
| a076 | Negative | Negative | True negative |
| a077 | Negative | Negative | True negative |
| a080 | Negative | Negative | True negative |
| a081 | Negative | Negative | True negative |
| a082 | Negative | Negative | True negative |
| a083 | Negative | Negative | True negative |
| a085 | Negative | Negative | True negative |
| a086 | Negative | Negative | True negative |
| a087 | Negative | Negative | True negative |
| a088 | Negative | Negative | True negative |
| a089 | Negative | Negative | True negative |
| a091 | Negative | Negative | True negative |
| a092 | Negative | Negative | True negative |
| a093 | Negative | Negative | True negative |
| a094 | Negative | Negative | True negative |
| a096 | Negative | Negative | True negative |
| a097 | Negative | Negative | True negative |
| a098 | Negative | Negative | True negative |
| a099 | Negative | Negative | True negative |
| a100 | Negative | Negative | True negative |
| a102 | Negative | Negative | True negative |
| a103 | Negative | Negative | True negative |
| a104 | Negative | Negative | True negative |
| a107 | Negative | Negative | True negative |
| a109 | Negative | Negative | True negative |
| a111 | Negative | Negative | True negative |
| a112 | Negative | Negative | True negative |
| a115 | Negative | Negative | True negative |
| a116 | Negative | Negative | True negative |
| a118 | Negative | Negative | True negative |
| a119 | Negative | Negative | True negative |
| a122 | Negative | Negative | True negative |
| a123 | Negative | Negative | True negative |
| a124 | Negative | Negative | True negative |
| a125 | Negative | Negative | True negative |
| a126 | Negative | Negative | True negative |
| a127 | Negative | Negative | True negative |
| a128 | Negative | Negative | True negative |
| a129 | Negative | Negative | True negative |
| a130 | Negative | Negative | True negative |
| a131 | Negative | Negative | True negative |
| a133 | Negative | Negative | True negative |
| a134 | Negative | Negative | True negative |
| a138 | Negative | Negative | True negative |
| a139 | Negative | Negative | True negative |
| a142 | Negative | Negative | True negative |
| a143 | Negative | Negative | True negative |
| a146 | Negative | Negative | True negative |
| a147 | Negative | Negative | True negative |
| a149 | Negative | Negative | True negative |
| a151 | Negative | Negative | True negative |
| a152 | Negative | Negative | True negative |
| a154 | Negative | Negative | True negative |
| a155 | Negative | Negative | True negative |
| a157 | Negative | Negative | True negative |
| a158 | Negative | Negative | True negative |
| a159 | Negative | Negative | True negative |
| a160 | Negative | Negative | True negative |
| a161 | Negative | Negative | True negative |
| a162 | Negative | Negative | True negative |
| a163 | Negative | Negative | True negative |
| a164 | Negative | Negative | True negative |
| a165 | Negative | Negative | True negative |
| a166 | Negative | Negative | True negative |
| a168 | Negative | Negative | True negative |
| a169 | Negative | Negative | True negative |
| a172 | Negative | Negative | True negative |
| a173 | Negative | Negative | True negative |
| a174 | Negative | Negative | True negative |
| a176 | Negative | Negative | True negative |
| a177 | Negative | Negative | True negative |
| a179 | Negative | Negative | True negative |
| a180 | Negative | Negative | True negative |
| a184 | Negative | Negative | True negative |
| a185 | Negative | Negative | True negative |
| a187 | Negative | Negative | True negative |
| a188 | Negative | Negative | True negative |
| a190 | Negative | Negative | True negative |
| a191 | Negative | Negative | True negative |
| a192 | Negative | Negative | True negative |
| a195 | Negative | Negative | True negative |
| a197 | Negative | Negative | True negative |
| a198 | Negative | Negative | True negative |
| a199 | Negative | Negative | True negative |
| a200 | Negative | Negative | True negative |
| a201 | Negative | Negative | True negative |
| a202 | Negative | Negative | True negative |
| a203 | Negative | Negative | True negative |
| a204 | Negative | Negative | True negative |
| a207 | Negative | Negative | True negative |
| a208 | Negative | Negative | True negative |
| a209 | Negative | Negative | True negative |
| a210 | Negative | Negative | True negative |
| a211 | Negative | Negative | True negative |
| a212 | Negative | Negative | True negative |
| a214 | Negative | Negative | True negative |
| a216 | Negative | Negative | True negative |
| a217 | Negative | Negative | True negative |
| a218 | Negative | Negative | True negative |
| a219 | Negative | Negative | True negative |
| a220 | Negative | Negative | True negative |
| a221 | Negative | Negative | True negative |
| a222 | Negative | Negative | True negative |
| a224 | Negative | Negative | True negative |
| a227 | Negative | Negative | True negative |
| a228 | Negative | Negative | True negative |
| a229 | Negative | Negative | True negative |
| a230 | Negative | Negative | True negative |
| a231 | Negative | Negative | True negative |
| a232 | Negative | Negative | True negative |
| a233 | Negative | Negative | True negative |
| a234 | Negative | Negative | True negative |
| a235 | Negative | Negative | True negative |
| a236 | Negative | Negative | True negative |
| a238 | Negative | Negative | True negative |
| a240 | Negative | Negative | True negative |
| a241 | Negative | Negative | True negative |
| a242 | Negative | Negative | True negative |
| a244 | Negative | Negative | True negative |
| a245 | Negative | Negative | True negative |
| a246 | Negative | Negative | True negative |
| a247 | Negative | Negative | True negative |
| a249 | Negative | Negative | True negative |
| a250 | Negative | Negative | True negative |
| a254 | Negative | Negative | True negative |
| a256 | Negative | Negative | True negative |
| a257 | Negative | Negative | True negative |
| a258 | Negative | Negative | True negative |
| a259 | Negative | Negative | True negative |
| a260 | Negative | Negative | True negative |
| a261 | Negative | Negative | True negative |
| a262 | Negative | Negative | True negative |
| a263 | Negative | Negative | True negative |
| a264 | Negative | Negative | True negative |
| a265 | Negative | Negative | True negative |
| a266 | Negative | Negative | True negative |
| a267 | Negative | Negative | True negative |
| a270 | Negative | Negative | True negative |
| a271 | Negative | Negative | True negative |
| a274 | Negative | Negative | True negative |
| a275 | Negative | Negative | True negative |
| a276 | Negative | Negative | True negative |
| a277 | Negative | Negative | True negative |
| a278 | Negative | Negative | True negative |
| a279 | Negative | Negative | True negative |
| a280 | Negative | Negative | True negative |
| a281 | Negative | Negative | True negative |
| a282 | Negative | Negative | True negative |
| a284 | Negative | Negative | True negative |
| a285 | Negative | Negative | True negative |
| a286 | Negative | Negative | True negative |
| a288 | Negative | Negative | True negative |
| a289 | Negative | Negative | True negative |
| a291 | Negative | Negative | True negative |
| a292 | Negative | Negative | True negative |
| a293 | Negative | Negative | True negative |
| a294 | Negative | Negative | True negative |
| a295 | Negative | Negative | True negative |
| a296 | Negative | Negative | True negative |
| a299 | Negative | Negative | True negative |
| a301 | Negative | Negative | True negative |
| a302 | Negative | Negative | True negative |
| a303 | Negative | Negative | True negative |
| a305 | Negative | Negative | True negative |
| a307 | Negative | Negative | True negative |
| a309 | Negative | Negative | True negative |
| a311 | Negative | Negative | True negative |
| a312 | Negative | Negative | True negative |
| a313 | Negative | Negative | True negative |
| a315 | Negative | Negative | True negative |
| a316 | Negative | Negative | True negative |
| a317 | Negative | Negative | True negative |
| a319 | Negative | Negative | True negative |
| a320 | Negative | Negative | True negative |
| a321 | Negative | Negative | True negative |
| a325 | Negative | Negative | True negative |
| a326 | Negative | Negative | True negative |
| a328 | Negative | Negative | True negative |
| a330 | Negative | Negative | True negative |
| a331 | Negative | Negative | True negative |
| a332 | Negative | Negative | True negative |
| a333 | Negative | Negative | True negative |
| a335 | Negative | Negative | True negative |
| a336 | Negative | Negative | True negative |
| a337 | Negative | Negative | True negative |
| a338 | Negative | Negative | True negative |
| a339 | Negative | Negative | True negative |
| a340 | Negative | Negative | True negative |
| a341 | Negative | Negative | True negative |
| a342 | Negative | Negative | True negative |
| a343 | Negative | Negative | True negative |
| a344 | Negative | Negative | True negative |
| a345 | Negative | Negative | True negative |
| a346 | Negative | Negative | True negative |
| a347 | Negative | Negative | True negative |
| a348 | Negative | Negative | True negative |
| b001 | Negative | Negative | True negative |
| b002 | Negative | Negative | True negative |
| b003 | Negative | Negative | True negative |
| b004 | Negative | Negative | True negative |
| b006 | Negative | Negative | True negative |
| b010 | Negative | Negative | True negative |
| b011 | Negative | Negative | True negative |
| b012 | Negative | Negative | True negative |
| b013 | Negative | Negative | True negative |
| b014 | Negative | Negative | True negative |
| b015 | Negative | Negative | True negative |
| b017 | Negative | Negative | True negative |
| b018 | Negative | Negative | True negative |
| b019 | Negative | Negative | True negative |
| b022 | Negative | Negative | True negative |
| b024 | Negative | Negative | True negative |
| b027 | Negative | Negative | True negative |
| b029 | Negative | Negative | True negative |
| b030 | Negative | Negative | True negative |
| b031 | Negative | Negative | True negative |
| b033 | Negative | Negative | True negative |
| b037 | Negative | Negative | True negative |
| b045 | Negative | Negative | True negative |
| b047 | Negative | Negative | True negative |
| b048 | Negative | Negative | True negative |
| b051 | Negative | Negative | True negative |
| b053 | Negative | Negative | True negative |
| b056 | Negative | Negative | True negative |
| b058 | Negative | Negative | True negative |
| b063 | Negative | Negative | True negative |
| b069 | Negative | Negative | True negative |
| b070 | Negative | Negative | True negative |
| b072 | Negative | Negative | True negative |
| b074 | Negative | Negative | True negative |
| b077 | Negative | Negative | True negative |
| b078 | Negative | Negative | True negative |
| b079 | Negative | Negative | True negative |
| b080 | Negative | Negative | True negative |
| b081 | Negative | Negative | True negative |
| b084 | Negative | Negative | True negative |
| b085 | Negative | Negative | True negative |
| b086 | Negative | Negative | True negative |
| b087 | Negative | Negative | True negative |
| b088 | Negative | Negative | True negative |
| b090 | Negative | Negative | True negative |
| b094 | Negative | Negative | True negative |
| b095 | Negative | Negative | True negative |
| b096 | Negative | Negative | True negative |
| b100 | Negative | Negative | True negative |
| b104 | Negative | Negative | True negative |
| b107 | Negative | Negative | True negative |
| b108 | Negative | Negative | True negative |
| b109 | Negative | Negative | True negative |
| b110 | Negative | Negative | True negative |
| b112 | Negative | Negative | True negative |
| b113 | Negative | Negative | True negative |
| b116 | Negative | Negative | True negative |
| b118 | Negative | Negative | True negative |
| b119 | Negative | Negative | True negative |
| b122 | Negative | Negative | True negative |
| b123 | Negative | Negative | True negative |
| b127 | Negative | Negative | True negative |
| b128 | Negative | Negative | True negative |
| b130 | Negative | Negative | True negative |
| b133 | Negative | Negative | True negative |
| b134 | Negative | Negative | True negative |
| b136 | Negative | Negative | True negative |
| b140 | Negative | Negative | True negative |
| b142 | Negative | Negative | True negative |
| b143 | Negative | Negative | True negative |
| b147 | Negative | Negative | True negative |
| b148 | Negative | Negative | True negative |
| b151 | Negative | Negative | True negative |
| b152 | Negative | Negative | True negative |
| b153 | Negative | Negative | True negative |
| b154 | Negative | Negative | True negative |
| b156 | Negative | Negative | True negative |
| b157 | Negative | Negative | True negative |
| b158 | Negative | Negative | True negative |
| b160 | Negative | Negative | True negative |
| b161 | Negative | Negative | True negative |
| b162 | Negative | Negative | True negative |
| b164 | Negative | Negative | True negative |
| b165 | Negative | Negative | True negative |
| b167 | Negative | Negative | True negative |
| b169 | Negative | Negative | True negative |
| b170 | Negative | Negative | True negative |
| b171 | Negative | Negative | True negative |
| b172 | Negative | Negative | True negative |
| b174 | Negative | Negative | True negative |
| b177 | Negative | Negative | True negative |
| b181 | Negative | Negative | True negative |
| b182 | Negative | Negative | True negative |
| b186 | Negative | Negative | True negative |
| b191 | Negative | Negative | True negative |
| b192 | Negative | Negative | True negative |
| b193 | Negative | Negative | True negative |
| b194 | Negative | Negative | True negative |
| b198 | Negative | Negative | True negative |

In the detection results, "del/dup" denotes copy number deletion/copy number duplication, "chrN" denotes chromosome N, "p/q" denotes short arm/long arm regions, "Mb" denotes a unit of copy number length in megabases, and "%" denotes a copy number abnormal rate.

As can be seen from Table 1, the false positive rate of the method for detecting chromosomal copy number variation provided in the example is 10/356 = 2.81%, which satisfies the requirements of CNV detection.

### Example II

A total of 178 nucleic acid samples to be tested were used, including 9 positive nucleic acid samples and 169 negative nucleic acid samples, which were obtained from cases tested using an NIPT product approved for medical device use.

The following Table 2 shows CNV detection results of the 178 nucleic acid samples to be tested provided in Example II of the present disclosure.

**Table 2**

| No. | True result | Detection result |
|---|---|---|
| M2-240207-010A-B07 | del(Yq11.222-q11.22 3,3.50M) | del(chrY:q11.222-q11.223,20820001-24500001,3.68 Mb,198.89%) |
| M2-240207-010A-G01 | del(6q13-q16.3,27.6 3M) | del(chr6:q14.1-q16.3,77260001-101940001,24.68M b,15.76%) |
| M2-240207-010A-G06 | del(4q21.23-q22.2,9. 36M) | del(chr4:q21.23-q22.3,85180001-95340001,10.16M b,17.03%);del(chr4:q23-q24,100180001-103700001 ,3.52Mb,17.87%) |
| M2-240222-010B-C07 | del(11p13-p12,7.03 M) | del(chr11:p13-p12,34600001-39220001,4.62Mb,23. 23%) |
| M2-240222-010B-D04 | del(Xp22.33-q28,150 .66M) | del(chrX:p22.33-p21.2,2700001-30460001,27.76Mb ,10.98%);del(chrX:p21.2-p11.22,30960001-5458000 1,23.62Mb,10.69%);del(chrX:q22.1-q22.3,10180000 1-104560001,2.76Mb,8.50%);del(chrX:q22.3-q24,10 6140001-120260001,14.12Mb,8.96%);del(chrX:q24-q28,120760001-153360001,32.60Mb,8.59%) |
| M2-240222-010B-E09 | del(5q14.3-q33.3,71. 37M) | del(chr5:q14.3-q15,90980001-97660001,6.68Mb,9. 59%);del(chr5:q22.3-q23.2,114240001-122660001, 8.42Mb,9.72%);del(chr5:q23.2-q31.1,125260001-13 5440001,10.18Mb,10.62%);del(chr5:q31.1-q32,136 140001-145940001,9.80Mb,9.34%);del(chr5:q32-q3 3.1,147340001-151140001,3.80Mb,9.89%) |
| M2-240222-010B-F01 | dup(2q33.2-q37.3,38 .58M) | dup(chr2:q33.2-q37.3,203580001-242840001,39.26 Mb,13.12%) |
| M2-240222-010B-F03 | del(18p11.32-p11.22 ,8.85M) | del(chr18:p11.32-p11.22,140001-8840001,8.70Mb, 20.60%) |
| M2-240222-010B-F08 | dup(18p11.32-p11.2 1,13.00M) | dup(chr18:p11.31-q11.1,3000001-18720001,15.72 Mb,16.98%) |
| M2-240207-010A-A07 | Negative | dup(chr5:q11.2-q12.1,56880001-59180001,2.30Mb ,11.43%) |
| M2-240207-010A-B09 | Negative | dup(chr4:q34.3,178000001-180000001,2.00Mb,13. 28%) |
| M2-240207-010A-C10 | Negative | del(chr7:q11.23,73140001-75160001,2.02Mb,6.99 %) |
| M2-240207-010A-C11 | Negative | del(chr2:q31.1,170700001-174020001,3.32Mb,6.02 %) |
| M2-240207-010A-E02 | Negative | del(chr5:p14.3-p14.1,20680001-26800001,6.12Mb,8 .50%) |
| M2-240207-010A-F01 | Negative | del(chr11:q12.3-q13.2,63220001-66420001,3.20Mb, 6.61%) |
| M2-240207-010A-F06 | Negative | dup(chr8:q24.21,127720001-129840001,2.12Mb,9.42 %) |
| M2-240207-010A-F07 | Negative | del(chr4:p14-p13,39320001-41320001,2.00Mb,8.12 %) |
| M2-240207-010A-G10 | Negative | dup(chr4:q34.3,178500001-181500001,3.00Mb,9.7 0%) |
| M2-240222-010B-B10 | Negative | dup(chr2:q21.1-q21.2,131680001-134200001,2.52 Mb,11.54%) |
| M2-240222-010B-C09 | Negative | dup(chr9:p23-p21.2,13320001-26980001,13.66Mb,1 4.76%) |
| M2-240222-010B-E12 | Negative | dup(chr8:p23.2,3780001-5780001,2.00Mb,10.87%) |
| M2-240207-010A-A01 | Negative | Negative |
| M2-240207-010A-A02 | Negative | Negative |
| M2-240207-010A-A03 | Negative | Negative |
| M2-240207-010A-A04 | Negative | Negative |
| M2-240207-010A-A05 | Negative | Negative |
| M2-240207-010A-A06 | Negative | Negative |
| M2-240207-010A-A08 | Negative | Negative |
| M2-240207-010A-A09 | Negative | Negative |
| M2-240207-010A-A10 | Negative | Negative |
| M2-240207-010A-A11 | Negative | Negative |
| M2-240207-010A-A12 | Negative | Negative |
| M2-240207-010A-B01 | Negative | Negative |
| M2-240207-010A-B02 | Negative | Negative |
| M2-240207-010A-B03 | Negative | Negative |
| M2-240207-010A-B04 | Negative | Negative |
| M2-240207-010A-B05 | Negative | Negative |
| M2-240207-010A-B06 | Negative | Negative |
| M2-240207-010A-B08 | Negative | Negative |
| M2-240207-010A-B10 | Negative | Negative |
| M2-240207-010A-B11 | Negative | Negative |
| M2-240207-010A-B12 | Negative | Negative |
| M2-240207-010A-C01 | Negative | Negative |
| M2-240207-010A-C02 | Negative | Negative |
| M2-240207-010A-C03 | Negative | Negative |
| M2-240207-010A-C04 | Negative | Negative |
| M2-240207-010A-C05 | Negative | Negative |
| M2-240207-010A-C06 | Negative | Negative |
| M2-240207-010A-C07 | Negative | Negative |
| M2-240207-010A-C08 | Negative | Negative |
| M2-240207-010A-C09 | Negative | Negative |
| M2-240207-010A-C12 | Negative | Negative |
| M2-240207-010A-D01 | Negative | Negative |
| M2-240207-010A-D02 | Negative | Negative |
| M2-240207-010A-D03 | Negative | Negative |
| M2-240207-010A-D04 | Negative | Negative |
| M2-240207-010A-D05 | Negative | Negative |
| M2-240207-010A-D06 | Negative | Negative |
| M2-240207-010A-D07 | Negative | Negative |
| M2-240207-010A-D08 | Negative | Negative |
| M2-240207-010A-D09 | Negative | Negative |
| M2-240207-010A-D10 | Negative | Negative |
| M2-240207-010A-D11 | Negative | Negative |
| M2-240207-010A-D12 | Negative | Negative |
| M2-240207-010A-E01 | Negative | Negative |
| M2-240207-010A-E03 | Negative | Negative |
| M2-240207-010A-E04 | Negative | Negative |
| M2-240207-010A-E05 | Negative | Negative |
| M2-240207-010A-E06 | Negative | Negative |
| M2-240207-010A-E07 | Negative | Negative |
| M2-240207-010A-E08 | Negative | Negative |
| M2-240207-010A-E09 | Negative | Negative |
| M2-240207-010A-E10 | Negative | Negative |
| M2-240207-010A-E11 | Negative | Negative |
| M2-240207-010A-E12 | Negative | Negative |
| M2-240207-010A-F02 | Negative | Negative |
| M2-240207-010A-F03 | Negative | Negative |
| M2-240207-010A-F04 | Negative | Negative |
| M2-240207-010A-F05 | Negative | Negative |
| M2-240207-010A-F08 | Negative | Negative |
| M2-240207-010A-F09 | Negative | Negative |
| M2-240207-010A-F10 | Negative | Negative |
| M2-240207-010A-F11 | Negative | Negative |
| M2-240207-010A-F12 | Negative | Negative |
| M2-240207-010A-G03 | Negative | Negative |
| M2-240207-010A-G05 | Negative | Negative |
| M2-240207-010A-G07 | Negative | Negative |
| M2-240207-010A-G08 | Negative | Negative |
| M2-240207-010A-G09 | Negative | Negative |
| M2-240207-010A-G11 | Negative | Negative |
| M2-240207-010A-G12 | Negative | Negative |
| M2-240207-010A-H01 | Negative | Negative |
| M2-240207-010A-HO2 | Negative | Negative |
| M2-240207-010A-H03 | Negative | Negative |
| M2-240207-010A-H04 | Negative | Negative |
| M2-240207-010A-H05 | Negative | Negative |
| M2-240207-010A-H06 | Negative | Negative |
| M2-240207-010A-H07 | Negative | Negative |
| M2-240207-010A-H08 | Negative | Negative |
| M2-240207-010A-H09 | Negative | Negative |
| M2-240222-010B-A01 | Negative | Negative |
| M2-240222-010B-A02 | Negative | Negative |
| M2-240222-010B-A03 | Negative | Negative |
| M2-240222-010B-A04 | Negative | Negative |
| M2-240222-010B-A05 | Negative | Negative |
| M2-240222-010B-A06 | Negative | Negative |
| M2-240222-010B-A07 | Negative | Negative |
| M2-240222-010B-A08 | Negative | Negative |
| M2-240222-010B-A09 | Negative | Negative |
| M2-240222-010B-A10 | Negative | Negative |
| M2-240222-010B-A11 | Negative | Negative |
| M2-240222-010B-A12 | Negative | Negative |
| M2-240222-010B-B01 | Negative | Negative |
| M2-240222-010B-B02 | Negative | Negative |
| M2-240222-010B-B03 | Negative | Negative |
| M2-240222-010B-B04 | Negative | Negative |
| M2-240222-010B-B05 | Negative | Negative |
| M2-240222-010B-B06 | Negative | Negative |
| M2-240222-010B-B07 | Negative | Negative |
| M2-240222-010B-B08 | Negative | Negative |
| M2-240222-010B-B09 | Negative | Negative |
| M2-240222-010B-B11 | Negative | Negative |
| M2-240222-010B-B12 | Negative | Negative |
| M2-240222-010B-C01 | Negative | Negative |
| M2-240222-010B-C02 | Negative | Negative |
| M2-240222-010B-C03 | Negative | Negative |
| M2-240222-010B-C04 | Negative | Negative |
| M2-240222-010B-C05 | Negative | Negative |
| M2-240222-010B-C06 | Negative | Negative |
| M2-240222-010B-C08 | Negative | Negative |
| M2-240222-010B-C10 | Negative | Negative |
| M2-240222-010B-C12 | Negative | Negative |
| M2-240222-010B-D02 | Negative | Negative |
| M2-240222-010B-D03 | Negative | Negative |
| M2-240222-010B-D07 | Negative | Negative |
| M2-240222-010B-D08 | Negative | Negative |
| M2-240222-010B-D09 | Negative | Negative |
| M2-240222-010B-D10 | Negative | Negative |
| M2-240222-010B-D11 | Negative | Negative |
| M2-240222-010B-D12 | Negative | Negative |
| M2-240222-010B-E01 | Negative | Negative |
| M2-240222-010B-E02 | Negative | Negative |
| M2-240222-010B-E03 | Negative | Negative |
| M2-240222-010B-E05 | Negative | Negative |
| M2-240222-010B-E06 | Negative | Negative |
| M2-240222-010B-E08 | Negative | Negative |
| M2-240222-010B-E10 | Negative | Negative |
| M2-240222-010B-E11 | Negative | Negative |
| M2-240222-010B-F02 | Negative | Negative |
| M2-240222-010B-F04 | Negative | Negative |
| M2-240222-010B-F06 | Negative | Negative |
| M2-240222-010B-F07 | Negative | Negative |
| M2-240222-010B-F09 | Negative | Negative |
| M2-240222-010B-F10 | Negative | Negative |
| M2-240222-010B-F11 | Negative | Negative |
| M2-240222-010B-F12 | Negative | Negative |
| M2-240222-010B-G02 | Negative | Negative |
| M2-240222-010B-G03 | Negative | Negative |
| M2-240222-010B-G04 | Negative | Negative |
| M2-240222-010B-G05 | Negative | Negative |
| M2-240222-010B-G06 | Negative | Negative |
| M2-240222-010B-G07 | Negative | Negative |
| M2-240222-010B-G08 | Negative | Negative |
| M2-240222-010B-G09 | Negative | Negative |
| M2-240222-010B-G10 | Negative | Negative |
| M2-240222-010B-G11 | Negative | Negative |
| M2-240222-010B-G12 | Negative | Negative |
| M2-240222-010B-H01 | Negative | Negative |
| M2-240222-010B-H02 | Negative | Negative |
| M2-240222-010B-H03 | Negative | Negative |
| M2-240222-010B-H04 | Negative | Negative |
| M2-240222-010B-H05 | Negative | Negative |
| M2-240222-010B-H06 | Negative | Negative |
| M2-240222-010B-H07 | Negative | Negative |
| M2-240222-010B-H08 | Negative | Negative |
| M2-240222-010B-H09 | Negative | Negative |
| M2-240222-010B-H10 | Negative | Negative |
| M2-240222-010B-H 11 | Negative | Negative |

As can be seen from Table 2, the positive detection rate of the method for detecting chromosomal copy number variation provided in the example is 100%, and the false positive rate is 12/178 = 6.74%, which satisfies the requirements of CNV detection.

The following Table 3 is a contingency table obtained by statistical analysis of Table 1 and Table 2.

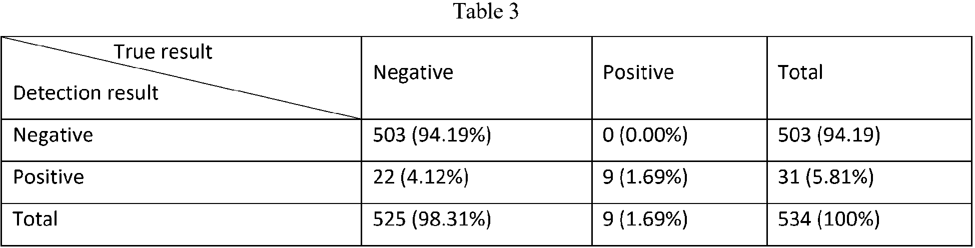

In the table, 503 represents the number of true negative samples, 0 represents the number of false negative samples, 22 represents the number of false positive samples, and 9 represents the number of true positive samples.

The following Table 4 is a summary table of performance indicators obtained by statistical analysis of Table 3.

**Table 4**

| Positive predictive value | Negative predictive value | Sensitivity | Specificity |
|---|---|---|---|
| 29.03% | 100% | 100% | 95.81% |

In the table, the positive predictive value represents a proportion of true positive samples among samples with positive detection results; the negative predictive value represents a proportion of true negative samples among samples with negative detection results; the sensitivity, also referred to as true positive rate, represents a proportion of actually positive samples that are correctly detected as positive; and the specificity, also referred to as true negative rate, represents a proportion of actually negative samples that are correctly detected as negative.

As can be seen from Tables 3 and 4, the method for detecting chromosomal copy number variation provided in the example has high accuracy.

The following is an embodiment of the apparatus for detecting chromosomal copy number variation according to the embodiments of the present disclosure. The apparatus and the method for detecting chromosomal copy number variation described in the above embodiments belong to the same inventive concept. For details that are not described in detail in the embodiment of the apparatus for detecting chromosomal copy number variation, reference may be made to the content of the method for detecting chromosomal copy number variation in the above embodiments.

FIG. 6 is a schematic structural diagram of an apparatus for detecting chromosomal copy number variation according to an embodiment of the present disclosure. As shown in FIG. 6, the apparatus includes: a sequencing depth data acquisition module 310, a copy number type set determination module 320, and a copy number variation region determination module 330.

The sequencing depth data acquisition module 310 is configured to obtain sequencing depth data of a chromosome to be tested in a nucleic acid sample to be tested, where the sequencing depth data include first sequencing depths under a plurality of first nucleic acid windows corresponding to the chromosome to be tested;
the copy number type set determination module 320 is configured to determine a copy number type set based on the sequencing depth data and a normal distribution function set corresponding to the chromosome to be tested, where the normal distribution function set includes normal distribution functions respectively corresponding to a copy number deletion type, a copy number duplication type, and a copy number normal type, and the copy number type set includes labeled copy number types of a plurality of second nucleic acid windows; and
the copy number variation region determination module 330 is configured to combine, based on the copy number type set, the plurality of second nucleic acid windows to obtain at least one copy number variation region on the chromosome to be tested,
where the second nucleic acid window is the first nucleic acid window, or the second nucleic acid window is a merged nucleic acid window obtained by merging the plurality of first nucleic acid windows based on a preset window number and a preset window step size, and the normal distribution function represents a probability density of a second sequencing depth corresponding to the second nucleic acid window varying with values of the second sequencing depth.

According to the technical solutions of the embodiments, a copy number type set corresponding to second nucleic acid windows is determined based on a normal distribution function set corresponding to a chromosome to be tested and sequencing depth data corresponding to first nucleic acid windows, and at least one copy number variation region on the chromosome to be tested is obtained by combining a plurality of second nucleic acid windows based on the copy number type set. The normal distribution function set includes normal distribution functions respectively corresponding to a copy number deletion type, a copy number duplication type, and a copy number normal type. The second nucleic acid window is the first nucleic acid window, or is a merged nucleic acid window obtained by merging a plurality of first nucleic acid windows based on a preset window number and a preset window step size. Therefore, the objective of performing CNV detection based on window fragment features of chromosomes is achieved, the problem of poor precision of CNV detection is solved, and the accuracy of CNV detection is improved.

In an optional embodiment, the copy number type set determination module 320 includes:
a normal distribution function set acquisition unit, configured to obtain a normal distribution function set in a first iteration process;
a labeled copy number type determination unit, configured to determine, for each second nucleic acid window, the second sequencing depth corresponding to the second nucleic acid window based on the sequencing depth data, and determine, based on the normal distribution function set and the second sequencing depth, probability density values respectively corresponding to the copy number deletion type, the copy number duplication type, and the copy number normal type of the second nucleic acid window, and use the copy number type corresponding to a maximum probability density value as the labeled copy number type of the second nucleic acid window;
a normal distribution function set updating unit, configured to update, based on the second sequencing depth of at least one second nucleic acid window corresponding to the copy number normal type, the normal distribution function set to obtain a normal distribution function set of a next iteration process;
a return execution unit, configured to return to execute the step of determining, for each second nucleic acid window, the second sequencing depth corresponding to the second nucleic acid window based on the sequencing depth data; and
a copy number type set determination unit, configured to, until the number of the second nucleic acid windows corresponding to the copy number normal type in the chromosome to be tested converges, determine the copy number type set based on the labeled copy number type of each second nucleic acid window in a current iteration process.

In an optional embodiment, the labeled copy number type determination unit is specifically configured to:
when the second nucleic acid window is a merged nucleic acid window, obtain first sequencing depths of a preset window number of first nucleic acid windows corresponding to the second nucleic acid window in the sequencing depth data; and
use an average value of the preset window number of first sequencing depths as the second sequencing depth corresponding to the second nucleic acid window.

In an optional embodiment, the normal distribution function set updating unit is specifically configured to:
determine a standard deviation corresponding to the second sequencing depth of at least one second nucleic acid window corresponding to the copy number normal type; and
update, based on the standard deviation, the normal distribution function set to obtain the normal distribution function set of the next iteration process.

In an optional embodiment, in the normal distribution function set, the mean value of the normal distribution function corresponding to the copy number normal type is 1, the mean value of the normal distribution function corresponding to the copy number deletion type is $1 - \frac{FF}{2}$, and the mean value of the normal distribution function corresponding to the copy number duplication type is $1 + \frac{FF}{2}$; or
in the normal distribution function set, the mean value of the normal distribution function corresponding to the copy number normal type is *mean,* the mean value of the normal distribution function corresponding to the copy number deletion type is $mean - \frac{FF}{2}$*,* and the mean value of the normal distribution function corresponding to the copy number duplication type is $mean + \frac{FF}{2}$*,*
where *mean* denotes a median value of the second sequencing depths respectively corresponding to a plurality of second nucleic acid windows, and *FF* denotes a placental-derived nucleic acid fraction corresponding to the nucleic acid sample to be tested.

In an optional embodiment, the copy number variation region satisfies the following combining conditions:
1) the labeled copy number types of a first second nucleic acid window and a last second nucleic acid window in the copy number variation region are both copy number abnormal types;
2) the number of second nucleic acid windows included in the copy number variation region whose labeled copy number types are the copy number abnormal types is not less than a preset number threshold;
3) the number of second nucleic acid windows included in the copy number variation region whose labeled copy number types correspond to the copy number normal type is not greater than a preset number threshold; and
4) a plurality of copy number abnormal types corresponding to the copy number variation region are either the copy number deletion type or the copy number duplication type.

In an optional embodiment, the apparatus further includes:
a copy number variation region screening module, configured to, after combining, based on the copy number type set, the plurality of second nucleic acid windows to obtain at least one copy number variation region on the chromosome to be tested, obtain, for each copy number variation region, copy number parameter data corresponding to the copy number variation region; and
in response to the copy number parameter data satisfying a parameter screening condition, use the copy number variation region as a final copy number variation region,
where the copy number parameter data include at least one of a copy number abnormal rate, a window density, and a region nucleic acid length, the copy number abnormal rate represents the ratio of the number of second nucleic acid windows having the copy number abnormal type in the copy number variation region to the total number of second nucleic acid windows in the copy number variation region, the window density represents the number of first nucleic acid windows included within a unit nucleic acid length, and the copy number abnormal type is the copy number deletion type or the copy number duplication type.

In an optional embodiment, the parameter screening condition includes at least one of the following: the copy number abnormal rate being greater than a preset abnormal rate threshold, the window density being greater than a preset density threshold, and the region nucleic acid length being greater than a preset length threshold.

In an optional embodiment, the sequencing depth data acquisition module 310 is specifically configured to:
obtain initial depth data of the chromosome to be tested in the nucleic acid sample to be tested, where the initial depth data include initial sequencing depths under a plurality of first nucleic acid windows corresponding to the chromosome to be tested;
obtain, for each first nucleic acid window, the initial sequencing depth corresponding to the first nucleic acid window and a reference sequencing depth, and use the ratio of the initial sequencing depth to the reference sequencing depth as the first sequencing depth corresponding to the first nucleic acid window; and
determine, based on the first sequencing depth corresponding to each first nucleic acid window, the sequencing depth data of the chromosome to be tested in the nucleic acid sample to be tested,
where the reference sequencing depth represents an average value of third sequencing depths respectively corresponding to the nucleic acid sample to be tested and at least one third nucleic acid window in a reference nucleic acid window set, and the third nucleic acid window is a nucleic acid window corresponding to other chromosomes except the chromosome to be tested.

In an optional embodiment, the apparatus further includes:
a reference depth data acquisition module, configured to obtain target depth data corresponding to the chromosome to be tested and reference depth data corresponding to the other chromosomes from a negative nucleic acid sample set, where the target depth data include target depth sequences under a plurality of first nucleic acid windows corresponding to the chromosome to be tested, the target depth sequences include negative sequencing depths of at least one negative nucleic acid sample in the negative nucleic acid sample set under the first nucleic acid window, the reference depth data include reference depth sequences under a plurality of third nucleic acid windows corresponding to the other chromosomes, and the reference depth sequences include negative sequencing depths of at least one negative nucleic acid sample under the third nucleic acid window; and
a reference nucleic acid window set determination module, configured to determine, for each first nucleic acid window, depth distance data based on the reference depth data and the target depth sequence corresponding to the first nucleic acid window, and screen, based on the depth distance data, the plurality of third nucleic acid windows to obtain the reference nucleic acid window set corresponding to the first nucleic acid window,
where the depth distance data include depth distances respectively corresponding to the target depth sequence and at least one reference depth sequence.

In an optional embodiment, the reference nucleic acid window set determination module includes:
an initial nucleic acid window set determination unit, configured to retain, based on the depth distance data, third nucleic acid windows satisfying a specified window number in ascending order to obtain an initial nucleic acid window set corresponding to the first nucleic acid window; and
a reference nucleic acid window set determination unit, configured to perform data cleaning on the initial nucleic acid window set to obtain the reference nucleic acid window set corresponding to the first nucleic acid window.

In an optional embodiment, the reference nucleic acid window set determination unit includes:
a first reference nucleic acid window set determination subunit, configured to, when the initial nucleic acid window set includes two adjacent third nucleic acid windows, obtain depth distances respectively corresponding to the two third nucleic acid windows in the depth distance data; and
remove, from the initial nucleic acid window set, the third nucleic acid window corresponding to the larger depth distance to obtain the reference nucleic acid window set corresponding to the first nucleic acid window.

In an optional embodiment, the reference nucleic acid window set determination unit includes:
a second reference nucleic acid window set determination subunit, configured to determine coefficient of variation data based on a reference depth sequence of each third nucleic acid window in the initial nucleic acid window set, where the coefficient of variation data include a coefficient of variation of each third nucleic acid window;
determine an outlier interval based on the coefficient of variation data; and
remove, from the initial nucleic acid window set, the third nucleic acid window corresponding to a coefficient of variation that does not satisfy the outlier interval in the coefficient of variation data to obtain the reference nucleic acid window set corresponding to the first nucleic acid window.

The apparatus for detecting chromosomal copy number variation according to the embodiments of the present disclosure can perform the method for detecting chromosomal copy number variation according to any embodiment of the present disclosure, and has corresponding functional modules for performing the method and beneficial effects.

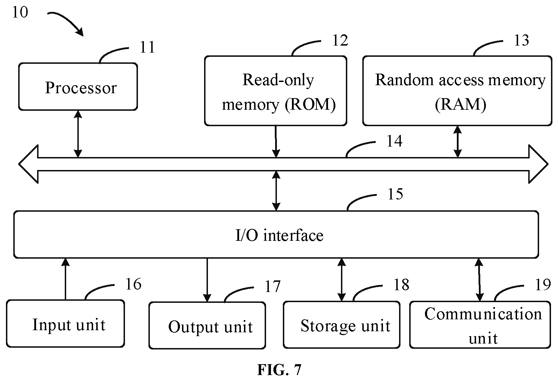

FIG. 7 is a schematic structural diagram of an electronic device according to an embodiment of the present disclosure. The electronic device 10 is intended to represent various forms of digital computers, such as a laptop, a desktop, a workstation, a server, a blade server, a mainframe, and other appropriate computers. The electronic device may also represent various forms of mobile apparatuses, such as a personal digital assistant, a cellular phone, a smart phone, a wearable device (such as a helmet, glasses, and a watch), and other similar computing apparatuses. The components shown herein, their connections and relationships, and their functions are merely exemplary and are not intended to limit the implementations of the present disclosure described and/or claimed herein.

As shown in FIG. 7, the electronic device 10 includes at least one processor 11 and a memory in communication connection to the at least one processor 11, such as a read-only memory (ROM) 12, or a random access memory (RAM) 13. The memory stores one or more computer programs that can be run by the at least one processor 11. The processor 11 can perform various appropriate actions and processes based on the computer program stored in the read-only memory (ROM) 12 or the computer program loaded from a storage unit 18 into the random access memory (RAM) 13. In the RAM 13, various programs and data necessary for the operation of the electronic device 10 may also be stored. The processor 11, the ROM 12, and the RAM 13 are connected to each other via a bus 14. An input/output (I/O) interface 15 is also connected to the bus 14.

A plurality of components in the electronic device 10 are connected to the I/O interface 15, including: an input unit 16, such as a keyboard or a mouse; an output unit 17, such as various types of displays or speakers; a storage unit 18, such as a magnetic disk or an optical disk; and a communication unit 19, such as a network card, a modem, or a wireless communication transceiver. The communication unit 19 allows the electronic device 10 to exchange information or data with other devices through a computer network such as the Internet and/or various telecommunication networks.

The processor 11 may be various general-purpose and/or dedicated processing components having processing and computing capabilities. Some examples of the processor 11 include, but are not limited to, a central processing unit (CPU), a graphics processing unit (GPU), various dedicated artificial intelligence (AI) computing chips, various processors running machine learning model algorithms, a digital signal processor (DSP), any appropriate processors, controllers, microcontrollers, and the like. The processor 11 performs the methods and processing described above, such as the method for detecting chromosomal copy number variation according to the above embodiments.

In some embodiments, the method for detecting chromosomal copy number variation according to the above embodiments can be implemented as a computer program, which is tangibly embodied in a computer-readable storage medium, such as the storage unit 18. In some embodiments, part or all of the computer program can be loaded and/or installed onto the electronic device 10 via the ROM 12 and/or the communication unit 19. When the computer program is loaded into the RAM 13 and run by the processor 11, one or more steps of the method for detecting chromosomal copy number variation described above can be performed. Alternatively, in other embodiments, the processor 11 may be configured to perform the method for detecting chromosomal copy number variation by any other suitable means (e.g., by means of firmware).

In particular, according to the embodiments of the present disclosure, the processes described above with reference to the flowcharts can be implemented as computer software programs. For example, the embodiments of the present disclosure include a computer program product including one or more computer programs carried on a non-transitory computer-readable medium, where the one or more computer programs include program codes for performing the methods shown in the flowcharts. In such embodiments, the one or more computer programs may be downloaded and installed from a network via the communication unit 19, or installed from the storage unit 18, or installed from the ROM 12. The one or more computer programs, when run by the processor 11, cause the processor to perform the above functions defined in the methods of the embodiments of the present disclosure.

Various embodiments of the systems and techniques described above herein may be implemented in the following systems or a combination thereof: digital electronic circuit systems, integrated circuit systems, field programmable gate arrays (FPGAs), application specific integrated circuits (ASICs), application specific standard parts (ASSPs), systems on chip (SoCs), complex programmable logic devices (CPLDs), computer hardware, firmware, software, and/or a combination thereof. These various embodiments may include: implementation in one or more computer programs, which can be run and/or interpreted on a programmable system including at least one programmable processor. The programmable processor may be of special or general purpose and capable of receiving data and instructions from, as well as transmitting data and instructions to, a storage system, at least one input apparatus, and at least one output apparatus.

The computer programs for implementing the method for detecting chromosomal copy number variation of the present disclosure may be written in any combination of one or more programming languages. The computer programs may be provided to the processor of a general-purpose computer, a special-purpose computer, or other programmable data processing apparatuses, such that the computer programs, when run by the processor, enable the implementation of the functions/operations specified in the flowcharts and/or block diagrams. The computer programs can be run entirely on the machine, partially on the machine, as a stand-alone software package partially on the machine and partially on a remote machine, or entirely on a remote machine or server.

In the context of the present application, the computer-readable storage medium may be a tangible medium, which may contain or store computer programs for use by, or in conjunction with, an instruction execution system, an apparatus, or a device. The computer-readable storage medium may include, but is not limited to, an electronic, magnetic, optical, electromagnetic, infrared, or semiconductor system, apparatus or device, or any suitable combination thereof. Alternatively, the computer-readable storage medium may be a machine-readable storage medium. Examples of the machine-readable storage medium may include an electrical connection based on at least one wire, a portable computer disk, a hard disk, a random access memory (RAM), a read-only memory (ROM), an erasable programmable read-only memory (EPROM), a flash memory, an optical fiber, a portable compact disc read-only memory (CD-ROM), an optical storage device, a magnetic storage device, or any suitable combination thereof.

To provide interaction with a user, the systems and techniques described herein may be implemented on a terminal device having: a display device for displaying information to the user (for example, a cathode-ray tube (CRT) or a liquid crystal display (LCD) monitor); and a keyboard and a pointing device (for example, a mouse or a trackball), through which the user can provide input to the terminal device. Other types of apparatuses may further be used to provide interaction with the user; for example, feedback provided to the user may be any form of sensory feedback (e.g., visual feedback, auditory feedback, or tactile feedback); and input from the user may be received in any form (including acoustic input, voice input, or tactile input).

The systems and techniques described herein may be implemented in a computing system that includes backend components (for example, as a data server), or in a computing system that includes middleware components (for example, an application server), or in a computing system that includes frontend components (for example, a user computer having a graphical user interface or a web browser, through which a user may interact with embodiments of the systems and techniques described herein), or in a computing system that includes any combination of such backend components, middleware components, or frontend components. The components of the system may be interconnected by any form or medium of digital data communication (for example, a communication network). Examples of communication networks include: a local area network (LAN), a wide area network (WAN), a blockchain network, and the Internet.

The computing system may include a client and a server. The client and the server are generally remote from each other and typically interact through a communication network. The client-server relationship is generated by computer programs running on the respective computers and having a client-server relationship with each other. The server may be a cloud server, also referred to as a cloud computing server or cloud host, which is a host product in a cloud computing service system, and is intended to overcome defects existing in conventional physical hosts and virtual private server (VPS) services, such as high management difficulty and weak service scalability.

It should be understood that the various forms of processes shown above may be reordered, augmented, or reduced in steps. For example, various steps described in the present disclosure may be performed in parallel, sequentially, or in different orders, which is not limited herein as long as the desired results of the technical solutions of the present disclosure can be achieved.

The above specific embodiments do not limit the protection scope of the present disclosure. It should be understood by those skilled in the art that various modifications, combinations, sub-combinations, and substitutions may be made according to design requirements and other factors. Any modification, equivalent replacement, or improvement made within the spirit and principle of the present disclosure should be included in the protection scope of the present disclosure.

## Claims

1. A method for detecting chromosomal copy number variation, comprising:
obtaining sequencing depth data of a chromosome to be tested in a nucleic acid sample to be tested, wherein the sequencing depth data comprise first sequencing depths under a plurality of first nucleic acid windows corresponding to the chromosome to be tested;
determining a copy number type set based on the sequencing depth data and a normal distribution function set corresponding to the chromosome to be tested, wherein the normal distribution function set comprises normal distribution functions respectively corresponding to a copy number deletion type, a copy number duplication type, and a copy number normal type, and the copy number type set comprises labeled copy number types of a plurality of second nucleic acid windows; and
combining, based on the copy number type set, the plurality of second nucleic acid windows to obtain at least one copy number variation region on the chromosome to be tested,
wherein the second nucleic acid window is the first nucleic acid window, or the second nucleic acid window is a merged nucleic acid window obtained by merging the plurality of first nucleic acid windows based on a preset window number and a preset window step size, and the normal distribution function represents a probability density of a second sequencing depth corresponding to the second nucleic acid window varying with values of the second sequencing depth.

2. The method according to claim 1, wherein determining the copy number type set based on the sequencing depth data and the normal distribution function set corresponding to the chromosome to be tested comprises:
obtaining a normal distribution function set in a first iteration process;
determining, for each second nucleic acid window, the second sequencing depth corresponding to the second nucleic acid window based on the sequencing depth data, and determining, based on the normal distribution function set and the second sequencing depth, probability density values respectively corresponding to the copy number deletion type, the copy number duplication type, and the copy number normal type of the second nucleic acid window, and using a copy number type corresponding to a maximum probability density value as the labeled copy number type of the second nucleic acid window; updating, based on the second sequencing depth of at least one second nucleic acid window corresponding to the copy number normal type, the normal distribution function set to obtain a normal distribution function set of a next iteration process;
returning to execute the step of determining, for each second nucleic acid window, the second sequencing depth corresponding to the second nucleic acid window based on the sequencing depth data; and
until a number of the second nucleic acid windows corresponding to the copy number normal type in the chromosome to be tested converges, determining the copy number type set based on the labeled copy number type of each second nucleic acid window in a current iteration process.

3. The method according to claim 2, wherein determining the second sequencing depth corresponding to the second nucleic acid window based on the sequencing depth data comprises:
when the second nucleic acid window is a merged nucleic acid window, obtaining first sequencing depths of a preset window number of first nucleic acid windows corresponding to the second nucleic acid window in the sequencing depth data; and
using an average value of the preset window number of first sequencing depths as the second sequencing depth corresponding to the second nucleic acid window.

4. The method according to claim 2, wherein updating, based on the second sequencing depth of at least one second nucleic acid window corresponding to the copy number normal type, the normal distribution function set to obtain the normal distribution function set of the next iteration process comprises:
determining a standard deviation corresponding to the second sequencing depth of at least one second nucleic acid window corresponding to the copy number normal type; and
updating, based on the standard deviation, the normal distribution function set to obtain the normal distribution function set of the next iteration process.

5. The method according to claim 2, wherein in the normal distribution function set, a mean value of the normal distribution function corresponding to the copy number normal type is 1, a mean value of the normal distribution function corresponding to the copy number deletion type is $1 - \frac{FF}{2}$, and a mean value of the normal distribution function corresponding to the copy number duplication type is $1 + \frac{FF}{2}$; or
in the normal distribution function set, a mean value of the normal distribution function corresponding to the copy number normal type is *mean,* a mean value of the normal distribution function corresponding to the copy number deletion type is $mean - \frac{FF}{2}$*,* and a mean value of the normal distribution function corresponding to the copy number duplication type is $mean + \frac{FF}{2}$*,*
wherein *mean* denotes a median value of the second sequencing depths respectively corresponding to a plurality of second nucleic acid windows, and *FF* denotes a placental-derived nucleic acid fraction corresponding to the nucleic acid sample to be tested.

6. The method according to any one of claims 1-5, wherein the copy number variation region satisfies the following combining conditions:
1) the labeled copy number types of a first second nucleic acid window and a last second nucleic acid window in the copy number variation region are both copy number abnormal types;
2) a number of second nucleic acid windows comprised in the copy number variation region whose labeled copy number types are the copy number abnormal types is not less than a preset number threshold;
3) a number of second nucleic acid windows comprised in the copy number variation region whose labeled copy number types correspond to the copy number normal type is not greater than a preset number threshold; and
4) a plurality of copy number abnormal types corresponding to the copy number variation region are either the copy number deletion type or the copy number duplication type.

7. The method according to any one of claims 1-6, wherein after combining, based on the copy number type set, the plurality of second nucleic acid windows to obtain at least one copy number variation region on the chromosome to be tested, the method further comprises:
obtaining, for each copy number variation region, copy number parameter data corresponding to the copy number variation region; and
in response to the copy number parameter data satisfying a parameter screening condition, using the copy number variation region as a final copy number variation region,
wherein the copy number parameter data comprise at least one of a copy number abnormal rate, a window density, and a region nucleic acid length, the copy number abnormal rate represents a ratio of the number of second nucleic acid windows having the copy number abnormal type in the copy number variation region to a total number of second nucleic acid windows in the copy number variation region, the window density represents a number of first nucleic acid windows comprised within a unit nucleic acid length, and the copy number abnormal type is the copy number deletion type or the copy number duplication type.

8. The method according to claim 7, wherein the parameter screening condition comprises at least one of the following: the copy number abnormal rate being greater than a preset abnormal rate threshold, the window density being greater than a preset density threshold, and the region nucleic acid length being greater than a preset length threshold.

9. The method according to any one of claims 1-8, wherein obtaining the sequencing depth data of the chromosome to be tested in the nucleic acid sample to be tested comprises:
obtaining initial depth data of the chromosome to be tested in the nucleic acid sample to be tested, wherein the initial depth data comprise initial sequencing depths under a plurality of first nucleic acid windows corresponding to the chromosome to be tested;
obtaining, for each first nucleic acid window, the initial sequencing depth corresponding to the first nucleic acid window and a reference sequencing depth, and using a ratio of the initial sequencing depth to the reference sequencing depth as the first sequencing depth corresponding to the first nucleic acid window; and
determining, based on the first sequencing depth corresponding to each first nucleic acid window, the sequencing depth data of the chromosome to be tested in the nucleic acid sample to be tested,
wherein the reference sequencing depth represents an average value of third sequencing depths respectively corresponding to the nucleic acid sample to be tested and at least one third nucleic acid window in a reference nucleic acid window set, and the third nucleic acid window is a nucleic acid window corresponding to other chromosomes except the chromosome to be tested.

10. The method according to claim 9, further comprising:
obtaining target depth data corresponding to the chromosome to be tested and reference depth data corresponding to the other chromosomes from a negative nucleic acid sample set, wherein the target depth data comprise target depth sequences under a plurality of first nucleic acid windows corresponding to the chromosome to be tested, the target depth sequences comprise negative sequencing depths of at least one negative nucleic acid sample in the negative nucleic acid sample set under the first nucleic acid window, the reference depth data comprise reference depth sequences under a plurality of third nucleic acid windows corresponding to the other chromosomes, and the reference depth sequences comprise negative sequencing depths of at least one negative nucleic acid sample under the third nucleic acid window; and
determining, for each first nucleic acid window, depth distance data based on the reference depth data and the target depth sequence corresponding to the first nucleic acid window, and screening, based on the depth distance data, the plurality of third nucleic acid windows to obtain the reference nucleic acid window set corresponding to the first nucleic acid window,
wherein the depth distance data comprise depth distances respectively corresponding to the target depth sequence and at least one reference depth sequence.

11. The method according to claim 10, wherein screening, based on the depth distance data, the plurality of third nucleic acid windows to obtain the reference nucleic acid window set corresponding to the first nucleic acid window comprises:
retaining, based on the depth distance data, third nucleic acid windows satisfying a specified window number in ascending order to obtain an initial nucleic acid window set corresponding to the first nucleic acid window; and
performing data cleaning on the initial nucleic acid window set to obtain the reference nucleic acid window set corresponding to the first nucleic acid window.

12. The method according to claim 11, wherein performing data cleaning on the initial nucleic acid window set to obtain the reference nucleic acid window set corresponding to the first nucleic acid window comprises:
when the initial nucleic acid window set comprises two adjacent third nucleic acid windows, obtaining depth distances respectively corresponding to the two third nucleic acid windows in the depth distance data; and
removing, from the initial nucleic acid window set, the third nucleic acid window corresponding to the larger depth distance to obtain the reference nucleic acid window set corresponding to the first nucleic acid window.

13. The method according to claim 11, wherein performing data cleaning on the initial nucleic acid window set to obtain the reference nucleic acid window set corresponding to the first nucleic acid window comprises:
determining coefficient of variation data based on a reference depth sequence of each third nucleic acid window in the initial nucleic acid window set, wherein the coefficient of variation data comprise a coefficient of variation of each third nucleic acid window;
determining an outlier interval based on the coefficient of variation data; and
removing, from the initial nucleic acid window set, the third nucleic acid window corresponding to a coefficient of variation that does not satisfy the outlier interval in the coefficient of variation data to obtain the reference nucleic acid window set corresponding to the first nucleic acid window.

14. An apparatus for detecting chromosomal copy number variation, comprising:
a sequencing depth data acquisition module, configured to obtain sequencing depth data of a chromosome to be tested in a nucleic acid sample to be tested, wherein the sequencing depth data comprise first sequencing depths under a plurality of first nucleic acid windows corresponding to the chromosome to be tested;
a copy number type set determination module, configured to determine a copy number type set based on the sequencing depth data and a normal distribution function set corresponding to the chromosome to be tested, wherein the normal distribution function set comprises normal distribution functions respectively corresponding to a copy number deletion type, a copy number duplication type, and a copy number normal type, and the copy number type set comprises labeled copy number types of a plurality of second nucleic acid windows; and
a copy number variation region determination module, configured to combine, based on the copy number type set, the plurality of second nucleic acid windows to obtain at least one copy number variation region on the chromosome to be tested,
wherein the second nucleic acid window is the first nucleic acid window, or the second nucleic acid window is a merged nucleic acid window obtained by merging the plurality of first nucleic acid windows based on a preset window number and a preset window step size, and the normal distribution function represents a probability density of a second sequencing depth corresponding to the second nucleic acid window varying with values of the second sequencing depth.

15. An electronic device, comprising:
at least one processor; and
a memory in communication connection to the at least one processor, wherein
the memory stores one or more computer programs run by the at least one processor, and the one or more computer programs, when run by the at least one processor, cause the at least one processor to perform the method for detecting chromosomal copy number variation according to any one of claims 1-13.
